# EUROPEAN PATENT SPECIFICATION

(11) **EP 4 525 621 B1**
(45) Date of publication and mention of the grant of the patent: **23.09.2026**
(21) Application number: 23840687.0
(22) Date of filing: 22.12.2023
(51) Int. Cl.: A01N 63/28, A01P 3/00, C12N 1/20

(54) **MEANS AND METHODS FOR CONTROLLING PATHOGENS AND PESTS IN PLANTS**
MITTEL UND VERFAHREN ZUR BEKÄMPFUNG VON PATHOGENEN UND SCHÄDLINGEN IN PFLANZEN
MOYENS ET PROCÉDÉS POUR LUTTER CONTRE DES PATHOGÈNES ET DES NUISIBLES DANS DES PLANTES

(30) Priority: 23.12.2022 EP 22216632
(43) Date of publication of application: 26.03.2025
(73) Proprietor: Aphea.Bio NV, 9052 Zwijnaarde (BE); Fundación Centro de Excelencia en Investigación de Medicamentos Innovadores en Andalucía, Medina, 18016 Granada (ES)
(72) Inventor: SIMON, Thomas, 9052 Zwijnaarde (BE); HOUBRAKEN, Michael, 9160 Lokeren (BE); DE MULDER, Thijs, 9031 Drongen (BE); GHODSALAVI, Behnoush, 9000 Gent (BE); VANDENABEELE, Steven, 9700 Oudenaarde (BE); VERCAUTEREN, Isabel, 9550 Herzele (BE); HAMONTS, Kelly, 9000 Gent (BE); IMPERATO, Valeria, 9000 Gent (BE); MAINTZ, Jens, 9052 Zwijnaarde (BE); LAGAE, Emma, 9000 Gent (BE)
(74) Representative: De Clercq & Partners
(86) International application number: PCT/EP2023/087608
(87) International publication number: WO 2024/133887

(56) References cited:
- CN-B- 104 726 362
- YOUSSEF Y. A. ET AL: "Plectosporium tabacinum Root Rot Disease of White Lupine (Lupinus termis Forsk.) and its Biological Control by Streptomyces Species", JOURNAL OF PHYTOPATHOLOGY - PHYTOPATHOLOGISCHE ZEITSCHRIFT., vol. 149, no. 1, 1 January 2001 (2001-01-01), DE, pages 29 - 33, XP093045324, ISSN: 0931-1785, DOI: 10.1046/j.1439-0434.2001.00565.x
- KIM HAE-RYOUNG ET AL: "Antifungal activity of Streptomyces costaricanus HR391 against some plant-pathogenic fungi", vol. 52, no. 4, 31 December 2016 (2016-12-31), KR, pages 437 - 443, XP093045345, ISSN: 0440-2413, Retrieved from the Internet <URL:https://koreascience.kr/article/JAKO201609633504863.pdf> DOI: 10.7845/kjm.2016.6056
- MINH NGUYEN VAN ET AL: "Control Efficacy of Streptomyces sp. A501 against Ginseng Damping-off and Its Antifungal Substance", MYBIOLOGY, vol. 45, no. 1, 19 March 2017 (2017-03-19), KR, pages 44 - 47, XP093045342, ISSN: 1229-8093, DOI: 10.5941/MYCO.2017.45.1.44
- HUMAN ZANDER R. ET AL: "Antifungal Streptomyces spp. Associated with the Infructescences of Protea spp. in South Africa", FRONTIERS IN MICROBIOLOGY, vol. 7, 2 November 2016 (2016-11-02), Lausanne, XP093045325, ISSN: 1664-302X, DOI: 10.3389/fmicb.2016.01657
- KOMAKI HISAYUKI: "Reclassification of Streptomyces costaricanus and Streptomyces phaeogriseichromatogenes as later heterotypic synonyms of Streptomyces murinus", vol. 71, no. 2, 1 February 2021 (2021-02-01), GB, pages 4638 - 10000001, XP093045330, ISSN: 1466-5026, Retrieved from the Internet <URL:https://www.microbiologyresearch.org/docserver/fulltext/ijsem/71/2/ijsem004638.pdf?expires=1683636260&id=id&accname=sgid024200&checksum=42485CA6D98CC68533FAD7D84AAEA5DF> DOI: 10.1099/ijsem.0.004638

## Description

### FIELD OF THE INVENTION

The present invention relates to the field of pest and pathogen control and resistance in plants using bacteria. The present invention discloses both means and methods for controlling these pests and pathogens.

### BACKGROUND

There is a continuous increase in the demand for agricultural products to be able to feed the growing human population as well as livestock. In addition, such growing demand puts a further strain on the environment, because of the conventional practices used in agriculture. The most serious problem encountered in the cultivation of crops is the loss of material and damage caused by harmful plant pathogens.

Various methods have been developed so far to control plant diseases and pests. Among them, the most used and the most developed method is the chemical control method using chemical pesticides. Chemical pesticides are convenient to use and have immediate effects to protect plants from pathogenic infections, but are listed as specified poisonous substances, poisonous substances, deleterious substance, etc., which are regulated by law. In recent years, the abuse of chemical pesticides has created social problems: intoxications and deaths caused by acute toxicity; contamination of food due to residual pesticides in agricultural products; and influence of the outflow of residual pesticides on the human body and environment. Furthermore, new pathogens and pests resistant to chemical pesticides are emerging, forcing the development of new types of pathogen control, creating an endless cycle.

A promising practice is the use of microorganisms for battling and controlling pathogenic plant infections and disease symptoms. US 7 037 879 for instance describes the use of endophytic bacteria to confer pest resistance of grass plants. CN 104726362 B and Y. A. Youssef et al in the Journal of Phytopathology - Phytopathologische Zeitschrift 2001, 149, pages 29-33 both teach *Streptomyces murinus* strains to control phytopathogenic fungi, and K. Hae-Ryoung et al in Misainmurhag Hoiji (Korean Journal of Microbiology) 2016, 52 (4) pages 437-443 teaches the use of a *S. costaricanus* strain (a later synonym for *S*. *murinus*) to control some phytopathogenic fungi.

Plants infected with pathogens have the above-mentioned resistance conferred by the endophytes. Therefore, no pesticides are required for cultivating these plants, which means that prevention is possible no matter what pathogenic occurrences have been predicted.

There is however a further need in the market for other bio-based pathogen and pest control methods and means, preferably broad-spectrum methods that are not harmful for the environment, the host, and the consumer. The current invention aims to provide a solution for the latter.

### SUMMARY OF THE INVENTION

The present invention and embodiments thereof serve to provide a solution to one or more of above-mentioned disadvantages. To this end, the present invention provides subject-matter as set forth in any one and all of (1) to (15) below:
(1) A purified bacterial strain suitable for controlling a fungal pathogen in a plant, wherein said strain is:
   - a strain as deposited with the Belgian Coordinated Collections of Micro-organisms as Deposit ID: LMG P-32901, or
   - a strain as deposited with the Polish Collection of Microorganisms as Deposit ID: B/00314, B/00229, or B/00431.
(2) The purified bacterial strain according to (1), wherein said strain is the bacterial strain as deposited as Deposit ID: LMG P-32901.
(3) The purified bacterial strain according to (1) or (2), wherein the fungal pathogen is selected from the group consisting of Fusarium graminearum, Zymoseptoria tritici, Puccinia striiformis, Alternaria solani, Botrytis cinerea, Fusarium oxysporum, Microdochium nivale, Monilinia fructigena, Penicilium digitatum, Penicillium expansum, Penicillium italicum, Pythium sulcatum, Rhizoctonia solani, Blumeria graminis, Podosphaera xanthii, Erysiphe necator, Oidium lycopersicum, and Sclerotinia sclerotiorum.
(4) An agricultural active formulation comprising the purified bacterial strain according to (1), (2) or (3), or comprising a microbial active ingredient that comprises a spore suspension, spray-dried spores or a whole cell broth derived from said bacterial strain as active ingredient, said formulation is suitable to be used as a fungal pathogen control agent for plants,
(5) An agricultural active formulation comprising a lysate derived from the bacterial strain according to (1), (2) or (3), wherein the lysate is obtained by a process comprising:
   a) growing said bacterial strain in a suitable culture medium under suitable growth conditions;
   b) obtaining the lysate by lysing the bacterial strain,
   said lysate is suitable to be used as a fungal pathogen control agent for plants.
(6) The agricultural active formulation according to (4) or (5), wherein said formulation further comprises an agricultural compatible excipient.
(7) A plant part coated with the agricultural active formulation according to (4), (5) or (6).
(8) Use of the purified bacterial strain according to (1), (2) or (3) or the agricultural formulation according to (4), (5) or (6), to control a fungal pathogen in plants, wherein said plant is preferably a crop.
(9) A method of controlling a fungal pathogen in plants which comprises artificially introducing the purified bacterial strain according to (1), (2) or (3), a bacterial population comprising said bacterial strain, or the agriculturally active formulation according to (4), (5) or (6) to a plant, a plant part or a substrate comprising or hosting said plant, thereby conferring fungal pathogen resistance or fungal pathogen control to said plant.
(10) The method of controlling a fungal pathogen in plants according to (9), wherein said pathogens are selected from pathogens belonging to Phytophthora infestans, Tilletia tritici, Claviceps purpurea, Oculimacula spp., Fusarium sp., Pytium sp., Erysiphe graminis, Erysiphe necator, Oidium lycopersicum, Puccinia graminis, Puccinia triticina, Puccinia striiformis, Pyrenophora tritici-repentis, Ramularia sp. (collo-cygni), Sclerotinia sclerotiorum, Botrytis cinerea, Rhizoctonia solani, Colletotrichum graminicola, Microdochium nivale, Gaeumannomyces graminis var. tritici, Tapesia sp., Thysanoptera, Ustilago spp. or Mycosphaerella spp., Alternaria solani, Monilinia fructigena, Blumeria graminis, Podosphaera xanthii, Penicillium digitatum, Penicillium expansum, or Penicillium italicum.
(11) The method of controlling a fungal pathogen in plants according to (9) or (10), wherein said plants are chosen from the group of Acer spp., Actinidia spp., Abelmoschus spp., Agave sisalana, Agropyron spp., Agrostis stolonifera, Allium spp., Amaranthus spp., Ammophila arenaria, Ananas comosus, Annona spp., Apium graveolens, Arachis spp., Artocarpus spp., Asparagus officinalis, Avena spp., Averrhoa carambola, Bambusa sp., Benincasa hispida, Bertholletia excelsea, Beta vulgaris, Brassica spp., Cadaba farinosa, Camellia sinensis, Canna indica, Cannabis sativa, Capsicum spp., Carex elata, Carica papaya, Carissa macrocarpa, Carya spp., Carthamus tinctorius, Castanea spp., Ceiba pentandra, Cichorium endivia, Cinnamomum spp., Citrullus lanatus, Citrus spp., Cocos spp., Coffea spp., Colocasia esculenta, Cola spp., Corchorus sp., Coriandrum sativum, Corylus spp., Crataegus spp., Crocus sativus, Cucurbita spp., Cucumis spp., Cynara spp., Daucus carota, Desmodium spp., Dimocarpus longan, Dioscorea spp., Diospyros spp., Echinochloa spp., Elaeis sp., Eleusine coracana, Eragrostis tef, Erianthus sp., Eriobotrya japonica, Eucalyptus sp., Eugenia uniflora, Fagopyrum spp., Fagus spp., Festuca arundinacea, Ficus carica, Fortunella spp., Fragaria spp., Ginkgo biloba, Glycine spp., Gossypium hirsutum, Helianthus spp., Hemerocallis fulva, Hibiscus spp., Hordeum spp., Ipomoea batatas, Juglans spp., Lactuca sativa, Lathyrus spp., Lens culinaris, Linum usitatissimum, Litchi chinensis, Lotus spp., Luffa acutangula, Lupinus spp., Luzula sylvatica, Lycopersicon spp., Macrotyloma spp., Malus spp., Malpighia emarginata, Mammea americana, Mangifera indica, Manihot spp., Manilkara zapota, Medicago sativa, Melilotus spp., Mentha spp., Miscanthus sinensis, Momordica spp., Morus nigra, Musa spp., Nicotiana spp., Olea spp., Opuntia spp., Ornithopus spp., Oryza spp., Panicum miliaceum, Panicum virgatum, Passiflora edulis, Pastinaca sativa, Pennisetum sp., Persea spp., Petroselinum crispum, Phalaris arundinacea, Phaseolus spp., Phleum pratense, Phoenix spp., Phragmites australis, Physalis spp., Pinus spp., Pistacia vera, Pisum spp., Poa spp., Populus spp., Prosopis spp., Prunus spp., Psidium spp., Punica granatum, Pyrus communis, Quercus spp., Raphanus sativus, Rheum rhabarbarum, Ribes spp., Ricinus communis, Rubus spp., Saccharum spp., Salix sp., Sambucus spp., Secale cereale, Sesamum spp., Sinapis sp., Solanum spp., Sorghum bicolor, Spinacia spp., Syzygium spp., Tagetes spp., Tamarindus indica, Theobroma cacao, Trifolium spp., Tripsacum dactyloides, Triticosecale rimpaui, Triticum spp., Tropaeolum minus, Tropaeolum majus, Vaccinium spp., Vicia spp., Vigna spp., Viola odorata, Vitis spp., Zea mays, Zizania palustris, Ziziphus spp.; including progenies and hybrids between the above.
(12) A method of producing a bacterial lysate suitable for controlling a fungal plant pathogen, wherein the method comprises the steps of:
   a) growing the purified bacterial strain according to (1), (2) or (3) in a suitable culture medium under suitable growth conditions; and
   b) obtaining the lysate by lysing the bacterial strain.
(13) A method of producing a bacterial supernatant suitable for controlling a fungal plant pathogen, the method comprising the steps of:
   a) growing the purified bacterial strain according to (1), (2) or (3) in a suitable culture medium; and
   b) removing the bacterial cells from the culture medium to obtain the supernatant.
(14) A method of producing an agricultural active formulation, the method comprising formulating the lysate produced according to (12) into an agricultural active formulation.
(15) A method of producing an agricultural active formulation, the method comprising the steps of:
   a) growing the purified bacterial strain according to (1), (2) or (3) in a suitable culture medium;
   b) removing the bacterial cells from the culture medium to obtain the supernatant; and
   c) formulating the supernatant produced in step b) into an agricultural active formulation.

### DEFINITIONS

Unless otherwise defined, all terms used herein, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refer to both singular and plural referents unless the context clearly dictates otherwise. By way of example, "a compartment" refers to one or more than one compartment.
"About" as used herein referring to a measurable value such as a parameter, an amount, a temporal duration, and the like, is meant to encompass variations of ±20% or less, preferably ±10% or less, more preferably ±5% or less, even more preferably ±1% or less, and still more preferably ±0.1% or less of and from the specified value, in so far such variations are appropriate to perform in the disclosed invention. However, it is to be understood that the value to which the modifier "about" refers is itself also specifically disclosed.
"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

The recitation of numerical ranges by endpoints includes all numbers and fractions subsumed within that range, as well as the recited endpoints.

Whereas the terms "one or more" or "at least one", such as one or more or at least one member(s) of a group of members, is clear *per se,* by means of further exemplification, the term encompasses *inter alia* a reference to any one of said members, or to any two or more of said members, such as, e.g., any ≥3, ≥4, ≥5, ≥6 or ≥7 etc. of said members, and up to all said members.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may.

The term "pathogen" as used herein relates to a causative agent of disease. Such causative agent can be, amongst others, a microorganism such as a virus, a bacterium, or a fungi. The term "pest" or "plant pest" refers to a destructive insect or another animal that attacks and (partially) destroys plants. Most important plant pests are insects, mites, nematodes, and gastropod molluscs. Plant pests may also transmit fungal, bacterial or viral infections. As used herein, pathogen and pest may be used interchangeably and may refer to both pathogens and pests as defined before.

The term "soil sourced" relates to an organism that originally originates from the soil, preferably from the root environment of plants and were sourced from the latter. Soil sourced organisms such as bacteria may then be further cloned or propagated in an environment outside their initial environment, such as a laboratory environment.

As used herein, the term "microorganism", "microbial strain" or "microbe" refers to any species or taxon of microorganism, including, but not limited to, archaea, bacteria, microalgae, fungi (including mold and yeast species), mycoplasmas, microspores, nanobacteria, oomycetes, and protozoa. A microbe or microorganism can encompass individual cells (e.g., unicellular microorganisms) or more than one cell (e.g., multi-cellular microorganism).

As used herein, the term "bacterium", "bacteria", or "bacterial" refers in general to any prokaryotic organism, and may reference an organism from either Kingdom Eubacteria (Bacteria), Kingdom Archaebacteria (Archaea), or both. In some cases, bacterial genera have been reassigned due to various reasons (such as, but not limited to, the evolving field of whole genome sequencing), and it is understood that such nomenclature reassignments are within the scope of any claimed genus.

The term "pathogen" or "plant pathogen" are known to be associated with the plant, its associated results, refers to organisms that cause harmful effects to the health and vigor of plants. Plant pathogens include fungi, bacteria, viruses, insects, nematodes, and the like.

The term "purified" is intended to specifically reference an organism, cell, tissue, polynucleotide, or polypeptide that is removed from its original source. The term "purified" does not necessarily reflect the extent to which the microbe has been purified.

As used herein, a "purified bacterial strain" is a bacterial strain that has been removed from its natural milieu. The term "purified bacterial strain" refers to substantially no other strains than the desired strain, and is therefore substantially free of other contaminants, which can include microbial contaminants. Further, as used herein, "purified bacterial strain" is intended to mean the strain separated from materials with which it is normally found in nature.

The terms "identity" or "identical", or "similarity" or "similar" in the context of nucleotide sequences refer to the nucleotides in the two sequences that are the same when aligned for maximum correspondence. There are different algorithms known in the art that can be used to measure nucleotide sequence identity.

Nucleotide sequence identity can be measured by a local or global alignment, preferably implementing an optimal local or optimal global alignment algorithm. For example, a global alignment may be generated using an implementation of the Needleman-Wunsch algorithm. For example, a local alignment may be generated using an implementation of the Smith-Waterman algorithm.

A gap is a region of an alignment wherein a sequence does not align to a position in the other sequence of the alignment. In global alignments, terminal gaps are discarded before identity is calculated. For both local and global alignments, internal gaps are counted as differences. A terminal gap is a region beginning at the end of a sequence in an alignment wherein the nucleotide in the terminal position of that sequence does not correspond to a nucleotide position in the other sequence of the alignment and extending for all contiguous positions in that sequence wherein the nucleotides of that sequence do not correspond to a nucleotide position in the other sequence of the alignment.

A "plant element" or "plant part" is intended to generically reference either a whole plant or a plant component, including but not limited to plant tissues, parts, and cell types. A plant element is preferably one of the following: whole plant, seedling, meristematic tissue, ground tissue, vascular tissue, dermal tissue, seed, leaf, root, shoot, stem, flower, ear, spike, spikelet, fruit, stolon, bulb, tuber, corm, keikis, bud, or fruit. As used herein, a "plant element" is synonymous to a "portion" of a plant, and refers to any part of the plant, and can include distinct tissues and/or organs, and may be used interchangeably with the term "tissue" throughout. In addition, a "plant element" is intended to generically reference any part of a plant that is able to initiate other plants via either sexual or asexual reproduction of that plant, for example but not limited to: seed, seedling, root, shoot, cutting, scion, graft, stolon, bulb, tuber, corm, keikis, or bud.

"Agricultural plants" or "plants of agronomic importance" include plants that are cultivated by humans for food, feed, fiber, fuel, and/or industrial purposes. In some embodiments, plants (including seeds and other plant elements) treated in accordance with the present invention are monocots and dicots. In a particular embodiment, the agricultural plant is selected from the group consisting of wheat (*Triticum aestivum* and related varieties), barley (*Hordeum vulgare* and related varieties), maize (*Zea mays* and related varieties), grapes, Solanaceae, Cucurbitaceae, soft fruit (e.g. strawberries, blueberries, raspberries, blackberries), stone fruit (e.g. peaches, nectarines), citrus, pome fruit.

An "active formulation" refers to a mixture of chemicals that facilitate the stability, storage, and/or application of the bacterial strain(s). Treatment formulations may comprise any one or more agents such as: a carrier, a solvent, an adjuvant, an oil, an emulsifier, a spreader, a cryoprotectant, a binder, a dispersant, a surfactant, a buffer, a tackifier, a microbial stabilizer, a fungicide, a complexing agent, an herbicide, a nematicide, an insecticide, a plant growth regulator, a rodenticide, a desiccant, a nutrient, an excipient, a wetting agent, or a salt.

As used herein an "agriculturally compatible carrier" or "agriculturally compatible excipient" refers to any material, other than water, that can be added to a plant element without causing or having an adverse effect on the plant element (e.g., reducing seed germination) or the plant that grows from the plant element, or the like.

As used herein, a "colony-forming unit" or "CFU" is used as a measure of viable microorganisms in a sample. A CFU is an individual viable cell capable of forming on a solid medium a visible colony whose individual cells are derived by cell division from one parental cell.

The term "supernatant" refers to the liquid broth remaining when cells grown in said broth are removed by centrifugation, filtration, sedimentation or other means well known in the art.

The term "extract" refers to various forms of microbial products. Said microbial products are obtained by removing the cell walls and/or cell membranes of the bacterial strains, a process known as lysis, thereby obtaining one or more endogenous products of the bacterial strains in culture.

### DESCRIPTION OF FIGURES

The following description of the figures of specific embodiments of the invention is merely exemplary in nature and is not intended to limit the present teachings, their application or uses.

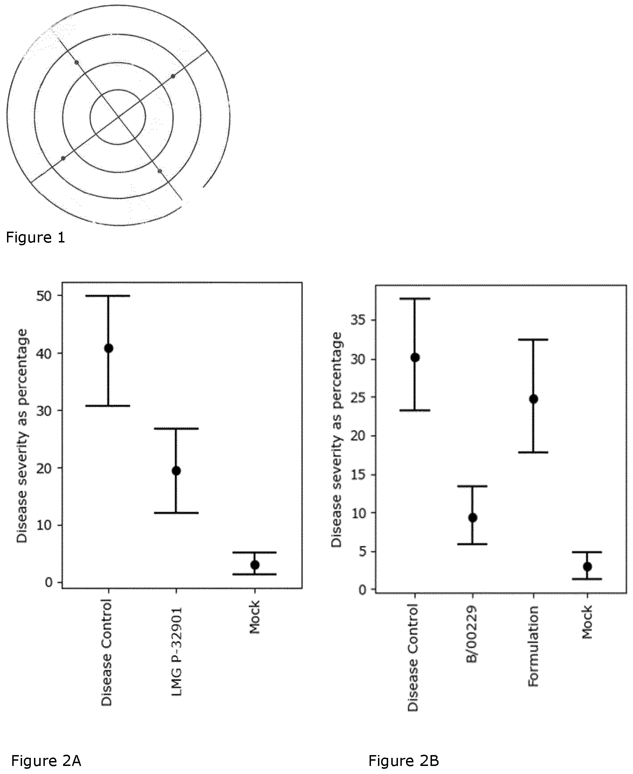

**Figure 1** shows a template used in co-culturing experiments with pathogenic fungi.

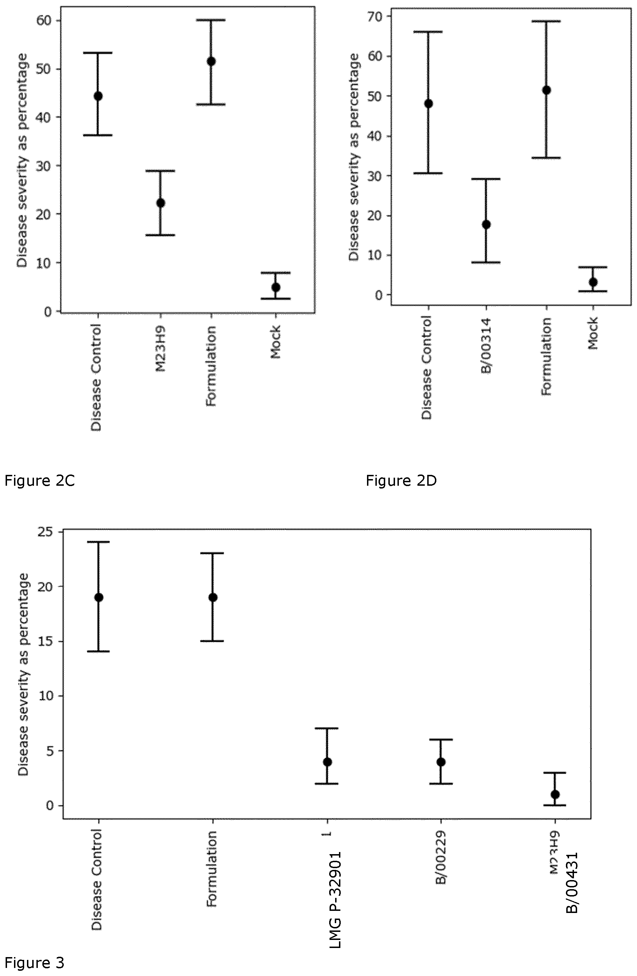

**Figures 2A-2D** show a graphical representation of the disease severity (% of necrosis) of wheat plants upon inoculation with the fungal pathogen *Fusarium graminearum* (Fg) compared to inoculation with Fg and an isolated bacterial strain according to the present invention, and compared to a Formulation control and Mock (a Formulation control with no pathogen applied later). Bacterial strain shown here have Deposit ID LMG P-32901 Fig. 2A); B/00229 (Fig. 2B); B/00431 (Fig. 2C); B/00314 (Fig. 2D).
**Figure 3** shows a graphical representation of the disease severity (% of necrosis) of wheat plants upon inoculation with the fungal pathogen *Puccinia striiformis var. tritici* (Pst) compared to inoculation with Pst and an isolated bacterial strain according to the present invention and compared to a Formulation control. Bacterial strain shown here have Deposit ID LMG P-32901, B/00229 and B/00431.

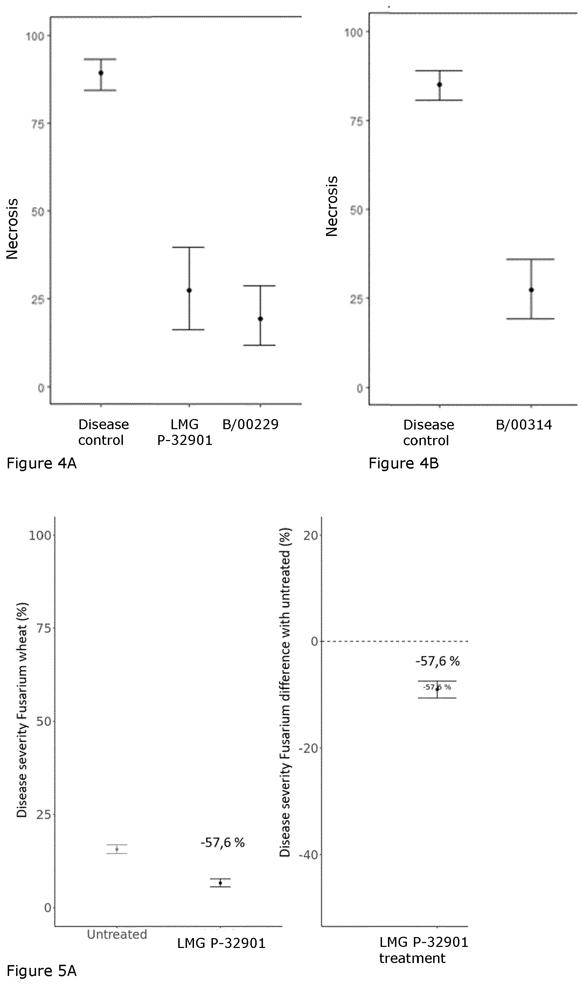

**Figure 4** shows a graphical representation of the disease severity (% of necrosis) of wheat plants upon inoculation with the fungal pathogen *Zymoseptoria tritici* compared to inoculation with Z. *tritici* and isolated bacterial strains according to the present invention. with Deposit ID LMG P-32901, B/00229 and B/00314, and compared to Mock treated wheat plants. Bacterial strain shown here have Deposit ID LMG P-32901 and B/00229 (Fig. 4A); B/00314 (Fig. 4B).
**Figure 5A** shows a graphical representation of the reduction of the fungal disease fusarium head blight severity caused by *Fusarium sp.* in *Triticum sp.* on a field in Poland in 2022. The figure on the left side visualizes on the Y-axis the severity of the disease for untreated wheat plants and wheat plants treated with the bacterial strain LMG P-32901. The figure on the right side shows the reduction of disease severity of wheat plants treated with the bacterial strain LMG P-32901 in comparison to untreated wheat plants.

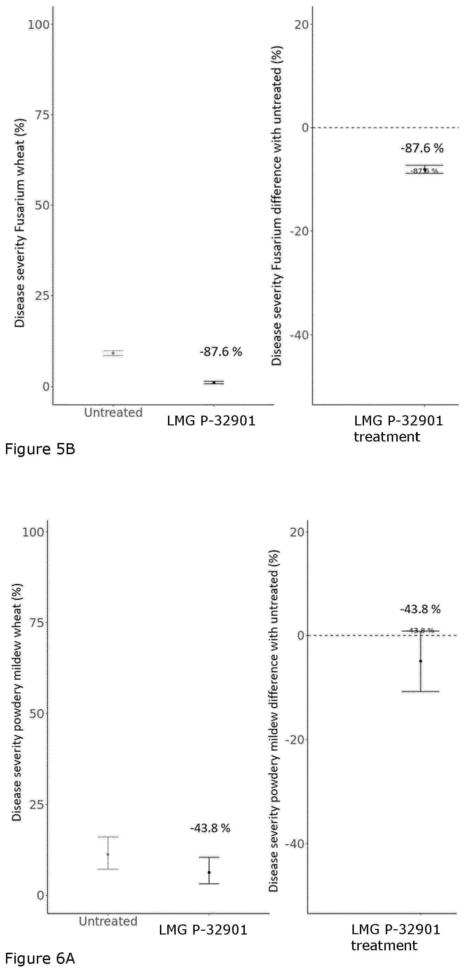

**Figure 5B** shows a graphical representation of the reduction of the fungal disease fusarium head blight severity caused by *Fusarium sp.* in *Triticum sp.* on a field in Bulgaria in 2022. The figure on the left side visualizes on the Y-axis the severity of the disease for untreated wheat plants and wheat plants treated with the bacterial strain LMG P-32901. The figure on the right side shows the reduction of disease severity of wheat plants treated with the bacterial strain LMG P-32901 in comparison to untreated wheat plants.
**Figure 6A** shows a graphical representation of the reduction of the fungal disease powdery mildew severity caused by *Blumeria graminis f. sp. tritici* in *Triticum sp.* on a field in France in 2022. The figure on the left side visualizes on the Y-axis the severity of the disease for untreated wheat plants and wheat plants treated with the bacterial strain LMG P-32901. The figure on the right side shows the reduction of disease severity of wheat plants treated with the bacterial strain LMG P-32901 in comparison to untreated wheat plants.

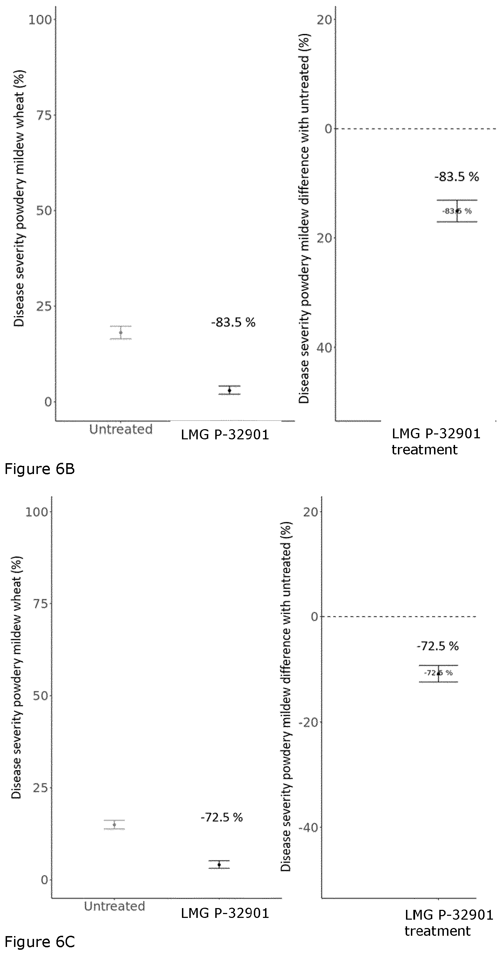

**Figure 6B** shows a graphical representation of the reduction of the fungal disease powdery mildew severity caused by *Blumeria graminis f. sp. tritici* in *Triticum sp.* on a field in Romania in 2022. The figure on the left side visualizes on the Y-axis the severity of the disease for untreated wheat plants and wheat plants treated with the bacterial strain LMG P-32901. The figure on the right side shows the reduction of disease severity of wheat plants treated with the bacterial strain LMG P-32901 in comparison to untreated wheat plants.
**Figure 6C** shows a graphical representation of the reduction of the fungal disease powdery mildew severity caused by *Blumeria graminis f. sp. tritici* in *Triticum sp.* on a field in Poland in 2022. The figure on the left side visualizes on the Y-axis the severity of the disease for untreated wheat plants and wheat plants treated with the bacterial strain LMG P-32901. The figure on the right side shows the reduction of disease severity of wheat plants treated with the bacterial strain LMG P-32901 in comparison to untreated wheat plants.

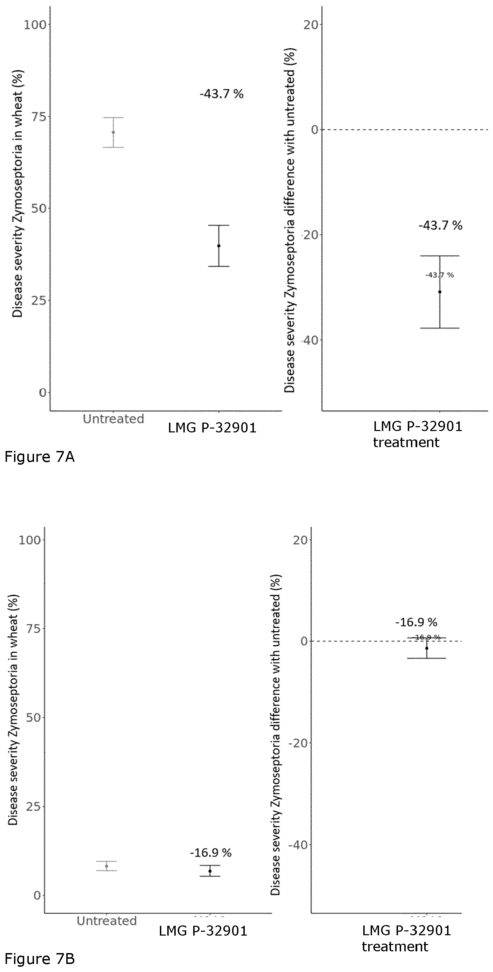

**Figure 7A** shows a graphical representation of the reduction of the fungal disease powdery mildew severity caused by *Zymoseptoria tritici* in *Triticum sp.* on a field in Poland in 2022. The figure on the left side visualizes on the Y-axis the severity of the disease for untreated wheat plants and wheat plants treated with the bacterial strain LMG P-32901. The figure on the right side shows the reduction of disease severity of wheat plants treated with the bacterial strain LMG P-32901 in comparison to untreated wheat plants.
**Figure 7B** shows a graphical representation of the reduction of the fungal disease powdery mildew severity caused by *Zymoseptoria tritici* in *Triticum sp.* on a field in Belgium in 2022. The figure on the left side visualizes on the Y-axis the severity of the disease for untreated wheat plants and wheat plants treated with the bacterial strain LMG P-32901. The figure on the right side shows the reduction of disease severity of wheat plants treated with the bacterial strain LMG P-32901 in comparison to untreated wheat plants.

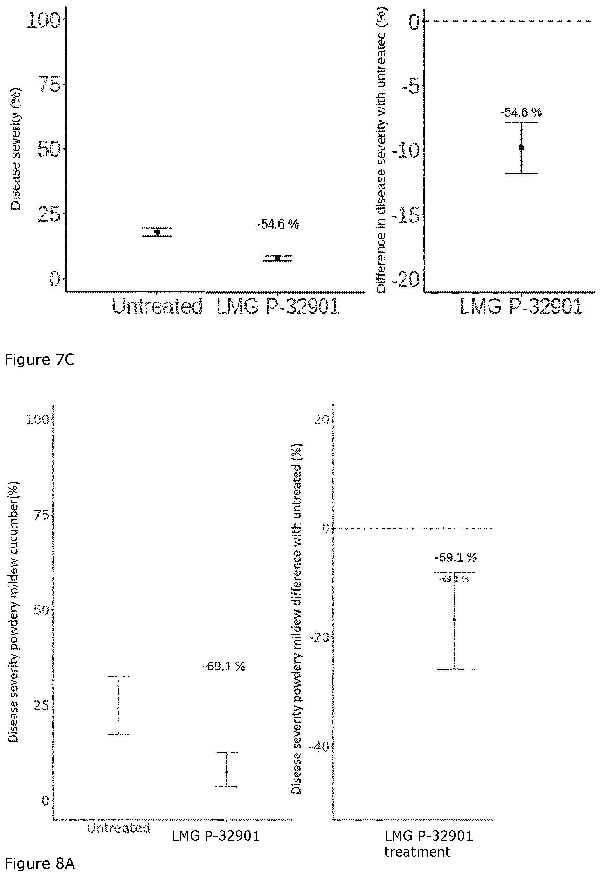

**Figure 7C** shows a graphical representation of the reduction of the fungal disease caused by *Zymoseptoria tritici* in *Triticum sp.* on a field in Germany in 2023. The figure on the left side visualizes on the Y-axis the severity of the disease for untreated wheat plants and wheat plants treated with the bacterial strain LMG P-32901. The figure on the right side shows the reduction of disease severity of wheat plants treated with the bacterial strain LMG P-32901 in comparison to untreated wheat plants.
**Figure 8A** shows a graphical representation of the reduction of the fungal disease powdery mildew severity caused by *Podosphaera xanthii* in *cucurbitaceae sp.* in a greenhouse in Italy in 2022. The figure on the left side visualizes on the Y-axis the severity of the disease for untreated cucumber plants and cucumber plants treated with the bacterial strain LMG P-32901. The figure on the right side shows the reduction of disease severity of cucumber plants treated with the bacterial strain LMG P-32901 in comparison to untreated cucumber plants.

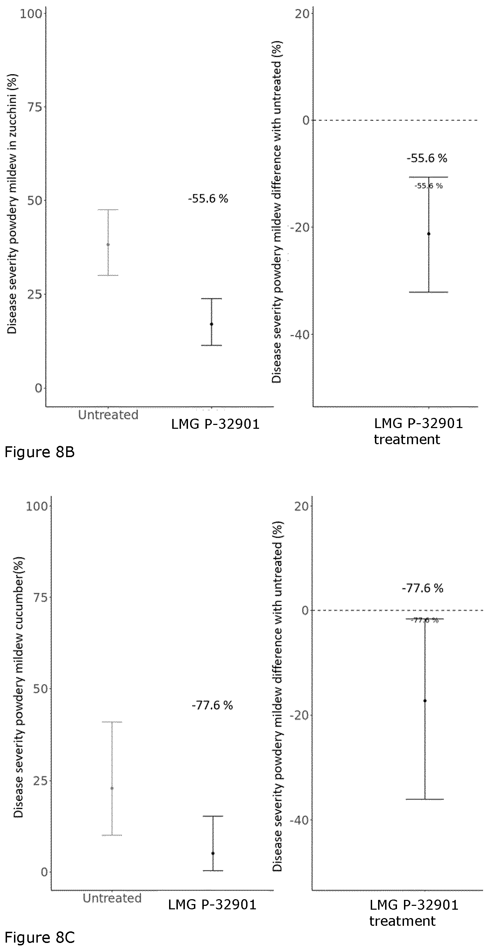

**Figure 8B** shows a graphical representation of the reduction of the fungal disease powdery mildew severity caused by *Podosphaera xanthii* in *cucurbitaceae sp.* in a field in Italy in 2022. The figure on the left side visualizes on the Y-axis the severity of the disease for untreated zucchini plants and zucchini plants treated with the bacterial strain LMG P-32901. The figure on the right side shows the reduction of disease severity of zucchini plants treated with the bacterial strain LMG P-32901 in comparison to untreated zucchini plants.
**Figure 8C** shows a graphical representation of the reduction of the fungal disease powdery mildew severity caused by *Podosphaera xanthii* in *cucurbitaceae sp.* in a greenhouse in Greece in 2022. The figure on the left side visualizes on the Y-axis the severity of the disease for untreated cucumber plants and cucumber plants treated with the bacterial strain LMG P-32901. The figure on the right side shows the reduction of disease severity of cucumber plants treated with the bacterial strain LMG P-32901 in comparison to untreated cucumber plants.

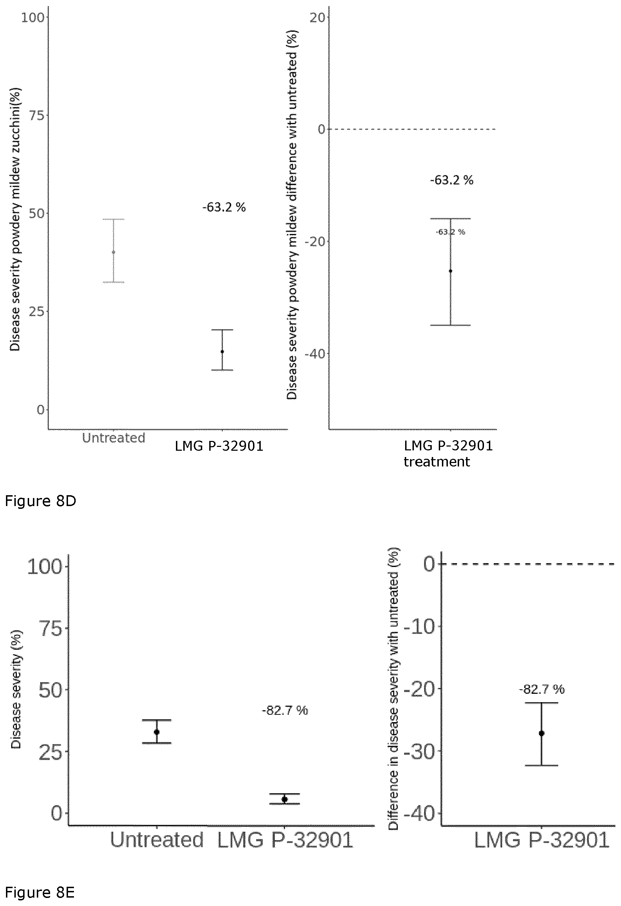

**Figure 8D** shows a graphical representation of the reduction of the fungal disease powdery mildew severity caused by *Podosphaera xanthii* in *cucurbitaceae sp.* in a field in Greece in 2022. The figure on the left side visualizes on the Y-axis the severity of the disease for untreated zucchini plants and zucchini plants treated with the bacterial strain LMG P-32901. The figure on the right side shows the reduction of disease severity of zucchini plants treated with the bacterial strain LMG P-32901 in comparison to untreated zucchini plants.
**Figure 8E** shows a graphical representation of the reduction of the fungal disease caused by *Podosphaera xanthii* in pumpkin on a field in Italy in 2023. The figure on the left side visualizes on the Y-axis the severity of the disease for untreated pumpkin plants and pumpkin plants treated with the bacterial strain LMG P-32901. The figure on the right side shows the reduction of disease severity of pumpkin plants treated with the bacterial strain LMG P-32901 in comparison to untreated pumpkin plants.

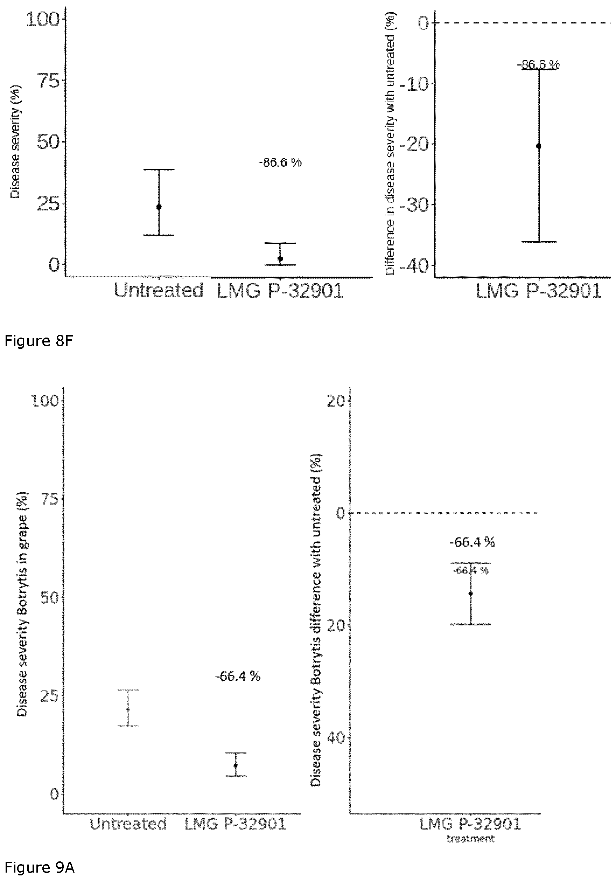

**Figure 8F** shows a graphical representation of the reduction of the fungal disease caused by *Podosphaera xanthii* in cucumber in a greenhouse in Spain in 2023. The figure on the left side visualizes on the Y-axis the severity of the disease for untreated cucumber plants and cucumber plants treated with the bacterial strain LMG P-32901. The figure on the right side shows the reduction of disease severity of cucumber plants treated with the bacterial strain LMG P-32901 in comparison to untreated cucumber plants.
**Figure 9A** shows a graphical representation of the reduction of the fungal disease grey mold severity caused by *Botrytis cinerea* in grapes in a field in Spain in 2022.
The figure on the left side visualizes on the Y-axis the severity of the disease for untreated bunches and bunches treated with the bacterial strain LMG P-32901. The figure on the right side shows the reduction of disease severity of bunches treated with the bacterial strain LMG P-32901 in comparison to untreated bunches.

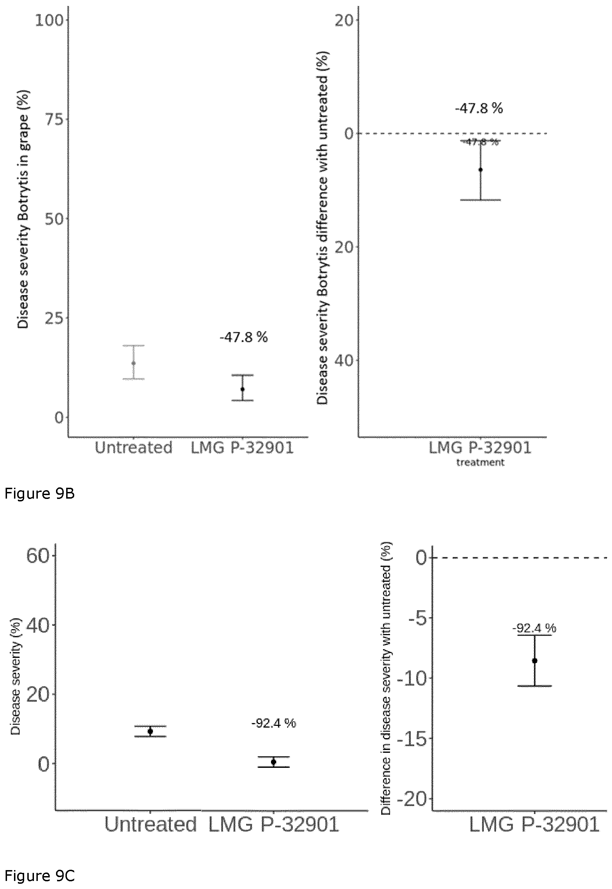

**Figure 9B** shows a graphical representation of the reduction of the fungal disease grey mold severity caused by *Botrytis cinerea* in grapes in a field in Spain in 2022.
The figure on the left side visualizes on the Y-axis the severity of the disease for untreated bunches and bunches treated with the bacterial strain LMG P-32901. The figure on the right side shows the reduction of disease severity of bunches treated with the bacterial strain LMG P-32901 in comparison to untreated bunches.
**Figure 9C** shows a graphical representation of the reduction of grey mold caused by *Botrytis cinerea* in wine grape in field conditions in Italy in 2023. The figure on the left side visualizes on the Y-axis the severity of the disease for untreated grapevine plants and grapevine plants treated with the bacterial strain LMG P-32901. The figure on the right side shows the reduction of disease severity of grapevine plants treated with the bacterial strain LMG P-32901 in comparison to untreated grapevine plants.

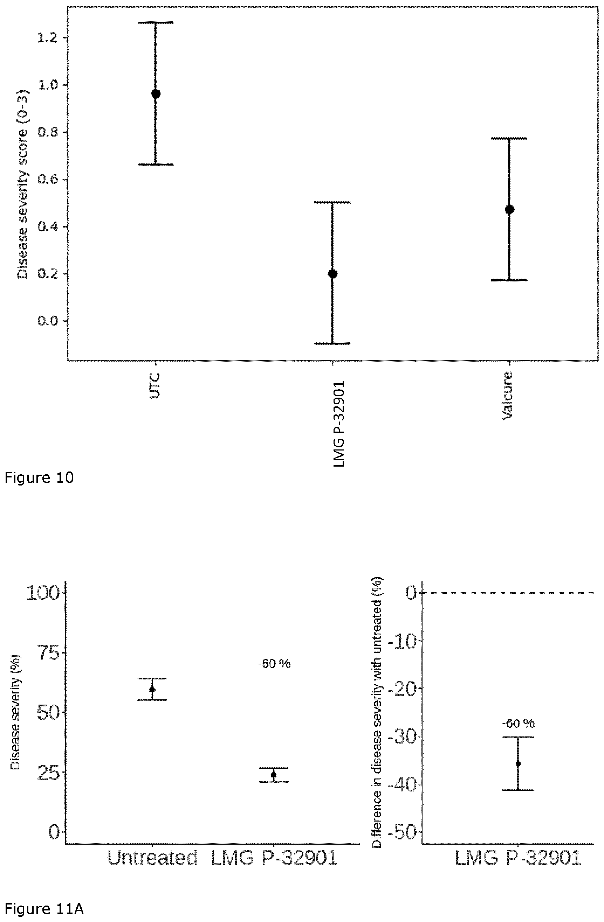

**Figure 10** shows a graphical representation of the reduction of the fungal disease caused by *Sclerotinia minor* in lettuce in a growth chamber trial in Italy in 2023. The figure visualizes on the Y-axis the disease severity score for the untreated lettuce plants, the lettuce plants treated with the bacterial strain LMG P-32901 and the reference product Valcure.
**Figure 11A** shows a graphical representation of the reduction of the fungal disease caused by *Puccinia striiformis var. tritici* in *Triticum sp.* on a field in Romania in 2023. The figure on the left side visualizes on the Y-axis the severity of the disease for untreated wheat plants and wheat plants treated with the bacterial strain LMG P-32901. The figure on the right side shows the reduction of disease severity of wheat plants treated with the bacterial strain LMG P-32901 in comparison to untreated wheat plants.

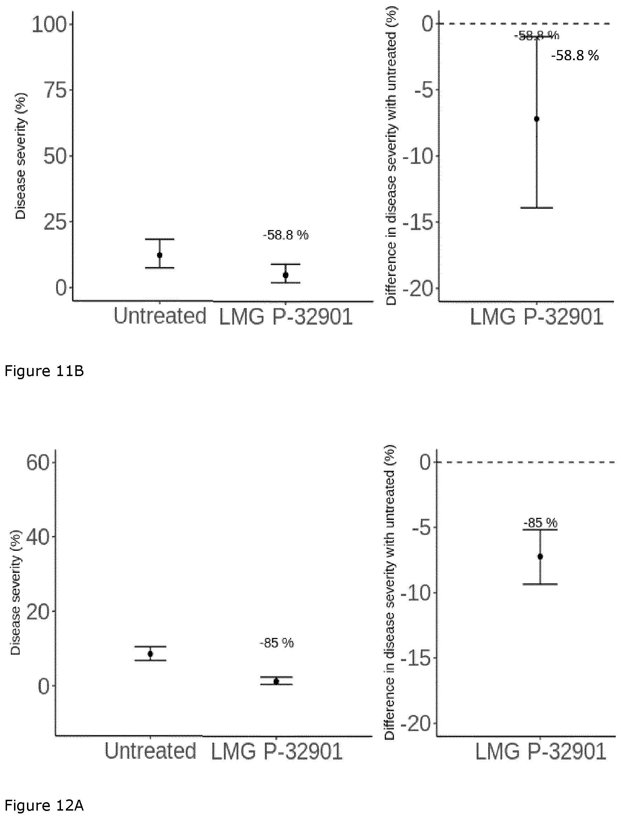

**Figure 11B** shows a graphical representation of the reduction of the fungal disease caused by *Puccinia striiformis var. tritici* in *Triticum sp.* on a field in Ireland in 2023. The figure on the left side visualizes on the Y-axis the severity of the disease for untreated wheat plants and wheat plants treated with the bacterial strain LMG P-32901. The figure on the right side shows the reduction of disease severity of wheat plants treated with the bacterial strain LMG P-32901 in comparison to untreated wheat plants.
**Figure 12A** shows a graphical representation of the reduction of grey mold caused by *Botrytis cinerea* in tomato in greenhouse conditions in Italy in 2023. The figure on the left side visualizes on the Y-axis the severity of the disease on leaves for untreated tomato plants and tomato plants treated with the bacterial strain LMG P-32901. The figure on the right side shows the reduction of disease severity on leaves of tomato plants treated with the bacterial strain LMG P-32901 in comparison to untreated tomato plants.

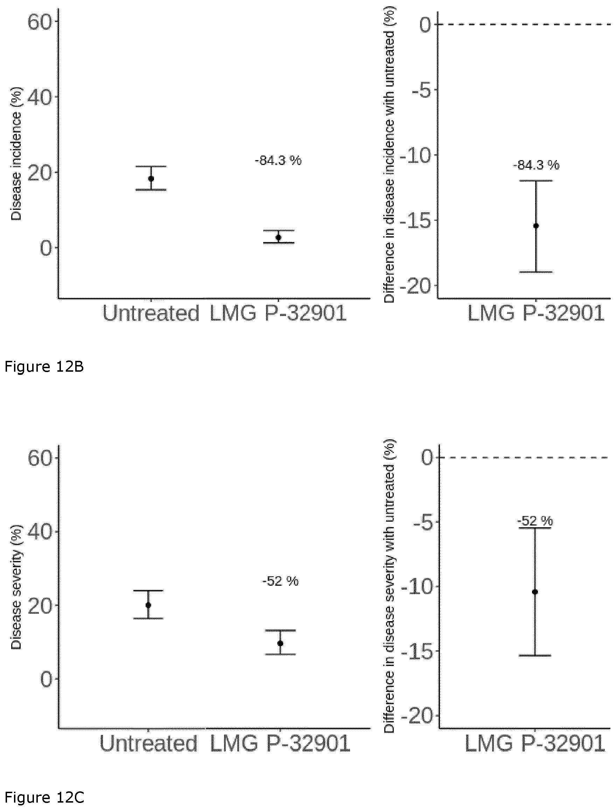

**Figure 12B** shows a graphical representation of the reduction of grey mold caused by *Botrytis cinerea* in tomato in greenhouse conditions in Italy in 2023. The figure on the left side visualizes on the Y-axis the incidence of the disease on fruits for untreated tomato plants and tomato plants treated with the bacterial strain LMG P-32901. The figure on the right side shows the reduction of disease incidence on fruits of tomato plants treated with the bacterial strain LMG P-32901 in comparison to untreated tomato plants.
**Figure 12C** shows a graphical representation of the reduction of grey mold caused by *Botrytis cinerea* in tomato in greenhouse conditions in Italy in 2023. The figure on the left side visualizes on the Y-axis the severity of the disease on leaves for untreated tomato plants and tomato plants treated with the bacterial strain LMG P-32901. The figure on the right side shows the reduction of disease severity on leaves of tomato plants treated with the bacterial strain LMG P-32901 in comparison to untreated tomato plants.

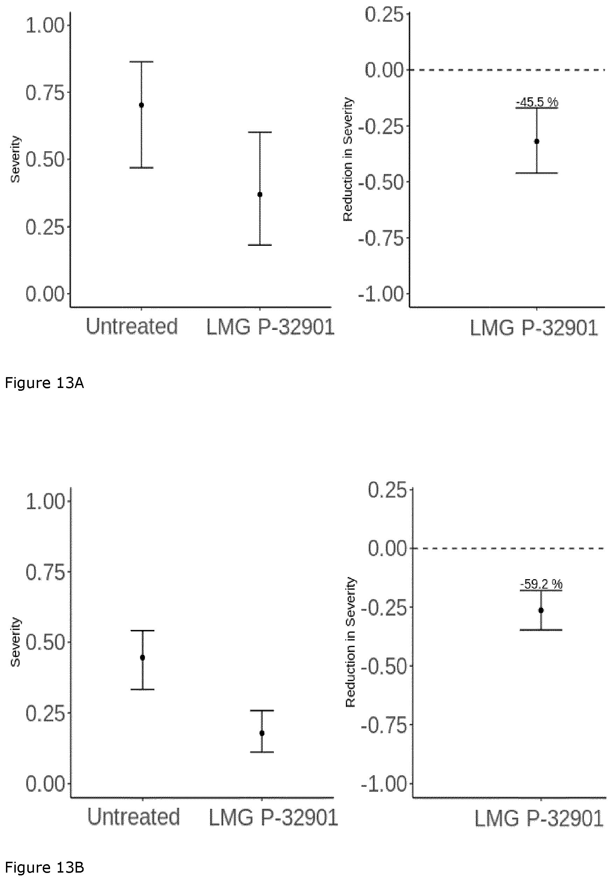

**Figure 13A** shows a graphical representation of the reduction of powdery mildew caused by *Uncinula necator* on bunches in grapevine in field conditions in Spain in 2023. The figure on the left side visualizes on the Y-axis the severity of the disease on fruits for untreated grapevine plants and grapevine plants treated with the bacterial strain LMG P-32901 (a severity score of 0-1 represents 0-100% disease severity). The figure on the right side shows the reduction of disease severity on fruits of grapevine plants treated with the bacterial strain LMG P-32901 in comparison to untreated grapevine plants.
**Figure 13B** shows a graphical representation of the reduction of powdery mildew caused by *Uncinula necator* on leaves in grapevine in field conditions in Spain in 2023. The figure on the left side visualizes on the Y-axis the severity of the disease on leaves for untreated grapevine plants and grapevine plants treated with the bacterial strain LMG P-32901 (a severity score of 0-1 represents 0-100% disease severity). The figure on the right side shows the reduction of disease severity on leaves of grapevine plants treated with the bacterial strain LMG P-32901 in comparison to untreated grapevine plants.

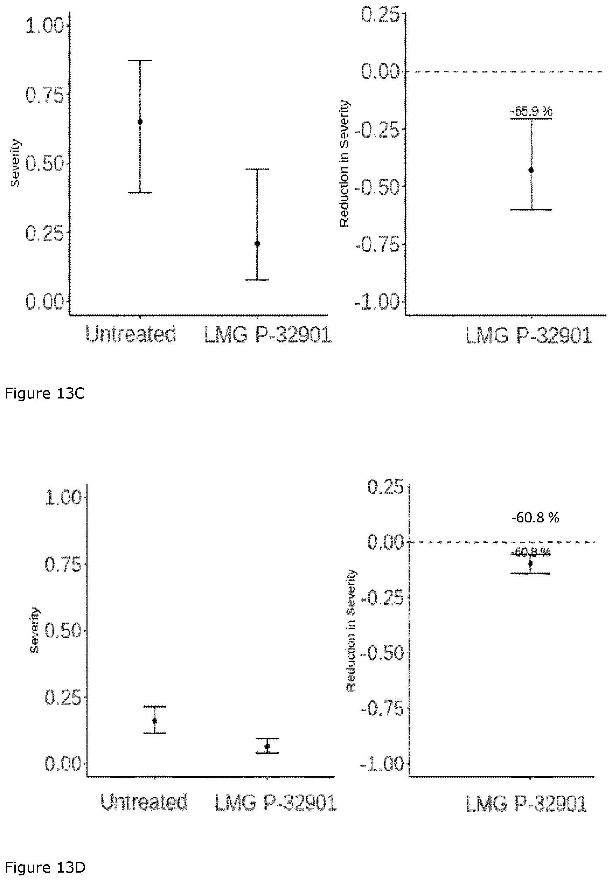

**Figure 13C** shows a graphical representation of the reduction of powdery mildew caused by *Uncinula necator* on bunches in grapevine in field conditions in Italy in 2023. The figure on the left side visualizes on the Y-axis the severity of the disease on fruits for untreated grapevine plants and grapevine plants treated with the bacterial strain (a severity score of 0-1 represents 0-100% disease severity). The figure on the right side shows the reduction of disease severity on fruits of grapevine plants treated with the bacterial strain LMG P-32901 in comparison to untreated grapevine plants.
**Figure 13D** shows a graphical representation of the reduction of powdery mildew caused by *Uncinula necator* on leaves in grapevine in field conditions in Italy in 2023. The figure on the left side visualizes on the Y-axis the severity of the disease on leaves for untreated grapevine plants and grapevine plants treated with the bacterial strain LMG P-32901 (a severity score of 0-1 represents 0-100% disease severity). The figure on the right side shows the reduction of disease severity on leaves of grapevine plants treated with the bacterial strain LMG P-32901 in comparison to untreated grapevine plants.

### DEPOSIT INFORMATION

The bacterial strains of the current invention were deposited under the terms of the Budapest Treaty.

Bacterial strain LMG P-32901 was deposited on 22 November 2022 with the Belgian Coordinated Collections of Micro-organisms (BCCM), Laboratorium voor Microbiologie - Bacteriënverzameling (LMG), Universiteit Gent, K.L. Ledeganckstraat 35, 9000 Gent, Belgium, having received Deposit ID: LMG P-32901.

Bacterial strain B/00229 was deposited on 21 August 2019, strain B/00314 was deposited on 7 October 2020, and strain B/00431 was deposited on 14 September 2022, all three with the Polish Collection of Microorganisms (Institute of Immunology and Experimental Therapy, Polish Academy of Sciences, Ul. Weigla 12, 53-114 Wroclaw, Poland), having received Deposit IDs: B/00229, B/00314, and B/00431 respectively.

The biological material shall be made available as provided for under Rule 13bis.6 PCT and Rule 32(1) EPC only by the issuance of a sample to an Expert.

### DETAILED DESCRIPTION OF THE INVENTION

The present disclosure concerns means and methods for controlling pests and/or pathogens in plants.

In a first aspect, this disclosure relates to a purified bacterial strain suitable for controlling a pest or a pathogen in a plant. Said strain is a strain of which a reference has been deposited with the Belgian Coordinated Collections of Micro-organisms as Deposit ID: LMG P-32901, with the Polish Collection of Microorganisms, respectively as Deposit ID: B/00314, B/00229, or B/00431. Also described herein, but not forming part of the invention, is a strain having at least 98 % genomic sequence identity with the bacterial strain as deposited as Deposit ID: LMG P-32901, B/00314, B/00229, or B/00431, and is still capable of controlling a pest or a pathogen in a plant. Particularly, said strain having at least 98 % genomic sequence identity with the deposited strains, preferably has at least 98.1 % genomic sequence identity with at least one of said deposited strains, more preferably at least 98.2 %, more preferably at least 98.3 %, more preferably at least 98.4 %, more preferably at least 98.5 %, more preferably at least 98.6 %, more preferably at least 98.7 %, more preferably at least 98.8 %, more preferably at least 98.9 %, more preferably at least 99 %, more preferably at least 99.1 %, more preferably at least 99.2 %, more preferably at least 99.3 %, more preferably at least 99.4 %, more preferably at least 99.5 %, more preferably at least 99.6 %, more preferably at least 99.7 %, more preferably at least 99.8 %, more preferably at least 99.9 % genomic sequence identity with a bacterial strain as deposited as Deposit ID: LMG P-32901, B/00314, B/00229, or B/00431, and is still capable of controlling a pest or pathogen in a plant.

To the purpose of using a strain herein disclosed for pest and/or pathogen control in plants, said strains may be applied to the plant or part of said plants as described below. In one example, said plant is Triticum, Secale, Hordeum, Avena, grapes, Solanaceae, Cucurbitaceae, soft fruit (e.g. strawberries, blueberries, raspberries, blackberries), stone fruit (e.g. peaches, nectarines), citrus or pome fruit.

In another example, said pests or pathogen may be any Eukaryotic or Prokaryotic pathogen or pests, such as but not limited to viral, bacterial, fungal pathogens or insect pests, as further described below.

The purified bacterial strains of the disclosure can be used in/as pathogen and/or pest control and/or to provide pest and/or pathogen resistance. Preferably, said pathogen control or resistance is effective both in vitro and in vivo.

In one example, said strain with at least 98 %, more preferably at least 98.1 %, more preferably at least 98.2 %, more preferably at least 98.3 %, more preferably at least 98.4 %, more preferably at least 98.5 %, more preferably at least 98.6 %, more preferably at least 98.7 %, more preferably at least 98.8 %, more preferably at least 98.9 %, more preferably at least 99 %, more preferably at least 99.1 %, more preferably at least 99.2 %, more preferably at least 99.3 %, more preferably at least 99.4 %, more preferably at least 99.5 %, more preferably at least 99.6 %, more preferably at least 99.7 %, more preferably at least 99.8 %, more preferably at least 99.9 % genomic sequence identity with a bacterial strain as deposited as Deposit ID: LMG 32901, B/00314, B/00229, or B/00431, is still capable of controlling in a plant the same pests or pathogens as reference strains LMG P-32901, B/00314, B/00229, or B/00431, preferably at least pests or pathogens chosen from the group of *Fusarium graminearum, Zymoseptoria tritici, Puccinia striiformis, Alternaria solani, Botrytis cinerea, Fusarium graminearum, Fusarium oxysporum, Microdochium nivale, Monilinia fructigena, Penicilium digitatum, Penicillium expansum, Penicillium italicum, Pythium sulcatum, Rhizoctonia solani, Sclerotinia sclerotiorum, Blumeria graminis, Podosphaera xanthii, Erysiphe necator, Oidium lycopersicum* or combinations thereof, but only the deposited strains LMG P-32901, B/00314, B/00229 and B/00431 form part of the invention.

In a second aspect, an agricultural active formulation is described comprising a purified bacterial strain as active ingredient, said formulation is suitable to be used as a pest or pathogen control agent for plants, wherein said bacterial strain is a purified bacterial strain as defined above.

Non-limiting examples of said agricultural active formulation are soluble powders, soluble granules, wettable granules, tablet formulations, dry flowables, aqueous flowables, wettable dispersible granules, oil dispersions, suspension concentrates, dispersible concentrates, emulsifiable concentrates, aqueous suspensions, a fertilizer granule, or a sprayable.

In one example, said formulation comprising a purified bacterial strain is a suspension concentrate-based formulation. Advantages thereof are that said type of formulation aids in stabilizing the bacterial strain, optionally in the form of spores, and optionally stabilizes any secondary metabolites produced by said strain.

In a further example, said purified bacterial strain is present at a concentration of at least about 10² CFU, cells or spores/ml in a liquid formulation; or at least about 10² CFU, cells or spores/mg in a non-liquid formulation. As non-limiting example, said cells may be living cells or spray-dried cells. Such enhanced concentrations of said bacterial strains are not found in nature. The bacterial strains have been propagated until they reached said concentrations.

Preferably, said purified bacterial strain is present at a CFU or spore concentration of at least 1 x 10^4 CFU or spores, at least 2 x 10^4 CFU or spores, at least 3 x 10^4 CFU or spores, at least 4 x 10^4 CFU or spores, at least 5 x 10^4 CFU or spores, at least 6 x 10^4 CFU or spores, at least 7 x 10^4 CFU or spores, at least 8 x 10^4 CFU or spores, at least 9 x 10^4 CFU or spores, at least 10 x 10^4 CFU or spores, at least 2 x 10^5 CFU or spores, at least 3 x 10^5 CFU or spores, at least 4 x 10^5 CFU or spores, at least 5 x 10^5 CFU or spores, at least 6 x 10^5 CFU or spores, at least 7 x 10^5 CFU or spores, at least 8 x 10^5 CFU or spores, at least 9 x 10^5 CFU or spores, at least 10 x 10^5 CFU or spores, at least 2 x 10^6 CFU or spores, at least 3 x 10^6 CFU or spores, at least 4 x 10^6 CFU or spores, at least 5 x 10^6 CFU or spores, at least 6 x 10^6 CFU or spores, at least 7 x 10^6 CFU or spores, at least 8 x 10^6 CFU or spores, at least 9 x 10^6 CFU or spores, at least 10 x 10^6 CFU or spores, at least 2 x 10^7 CFU or spores, at least 3 x 10^7 CFU or spores, at least 4 x 10^7 CFU or spores, at least 5 x 10^7 CFU or spores, at least 6 x 10^7 CFU or spores, at least 7 x 10^7 CFU or spores, at least 8 x 10^7 CFU or spores, at least 9 x 10^7 CFU or spores, at least 10 x 10^7 CFU or spores, at least 2 x 10^8 CFU or spores, at least 3 x 10^8 CFU or spores, at least 4 x 10^8 CFU or spores, at least 5 x 10^8 CFU or spores, at least 6 x 10^8 CFU or spores, at least 7 x 10^8 CFU or spores, at least 8 x 10^8 CFU or spores, at least 9 x 10^8 CFU or spores, at least 10 x 10^8 CFU or spores, at least 2 x 10^9 CFU or spores, at least 3 x 10^9 CFU or spores, at least 4 x 10^9 CFU or spores, at least 5 x 10^9 CFU or spores, at least 6 x 10^9 CFU or spores, at least 7 x 10^9 CFU or spores, at least 8 x 10^9 CFU or spores, at least 9 x 10^9 CFU or spores, at least 10 x 10^9 CFU or spores per ml when in a liquid formation or per gram when in a non-liquid formation.

More preferably, said purified bacterial strain is present at a CFU or spore concentration of between 10 x 10^4 and 10 x 10^9 CFU or spores, between 2 x 10^5 and 9 x 10^9 CFU or spores, between 3 x 10^5 and 8 x 10^9 CFU or spores, between 4 x 10^5 and 7 x 10^9 CFU or spores, between 5 x 10^5 and 6 x 10^9 CFU or spores, between 6 x 10^5 and 5 x 10^9 CFU or spores, between 7 x 10^5 and 4 x 10^9 CFU or spores, between 8 x 10^5 and 3 x 10^9 CFU or spores, between 9 x 10^5 and 2 x 10^9 CFU or spores, between 10 x 10^5 and 10 x 10^8 CFU or spores. More preferably between 2 x 10^6 and 10 x 10^8 CFU or spores, between 3 x 10^6 and 10 x 10^8 CFU or spores, between 4 x 10^6 and 10 x 10^8 CFU or spores, between 5 x 10^6 and 10 x 10^8 CFU or spores, between 6 x 10^6 and 10 x 10^8 CFU or spores, between 7 x 10^6 and 10 x 10^8 CFU or spores, between 8 x 10^6 and 10 x 10^8 CFU or spores, between 9 x 10^6 and 10 x 10^8 CFU or spores, between 10 x 10^6 and 10 x 10^8 CFU or spores per ml when in a liquid formation or per gram when in a non-liquid formation. Even more preferably between 2 x 10^7 and 10 x 10^8 CFU or spores, between 3 x 10^7 and 10 x 10^8 CFU or spores, between 4 x 10^7 and 10 x 10^8 CFU or spores, between 5 x 10^7 and 10 x 10^8 CFU or spores, between 6 x 10^7 and 10 x 10^8 CFU or spores, between 7 x 10^7 and 10 x 10^8 CFU or spores, between 8 x 10^7 and 10 x 10^8 CFU or spores, between 9 x 10^7 and 10 x 10^8 CFU or spores per ml when in a liquid formation or per gram when in a non-liquid formulation.

In another or further example, said formulation comprises one or more bacterial strains as disclosed herein as a mixture. In an example, said formulation comprises at least 2, at least 3, or at least 4 bacterial strains as disclosed herein or any suitable mixture. Said combination of strains preferably has a synergistic effect in controlling (a) pest(s) or pathogen(s) in a plant. When more than one bacterial strain is present in the formulation, said one or more purified bacterial strains are present at a concentration of at least about 10² CFU, cells or spores/ml per strain or for the total of said strains in a liquid formulation, or any of the concentrations as described above; or at least about 10² CFU, cells or spores/mg per strain or for the total of said strains in a non-liquid formulation, or any of the concentrations as described above.

In another example, said formulation is formulated to a high colony forming units (CFU), cells, or spores of the purified bacterial strain. The CFU concentration of the formulation is higher than the concentration at which the bacteria would exist naturally. Said CFU, cell or spore concentration is effective against plant pathogens and pests.

In another example, besides its effect against pests or pathogens, the concentration of said one or more bacterial strains or of one or more microbial active ingredients in the formulation is further effective to improve the plant growth and/or yield. In another example, said agricultural active formulation further comprises at least one oil, surfactant, and/or polymer, preferably at least one oil, one surfactant and one polymer. Preferably, said formulation further comprises one or more of the following: fungicide, nematicide, bactericide, insecticide, molluscicide, algicide, herbicide, fertilizer, micronutrient fertilizer material, stabilizer, preservative, carrier or excipient, complexing agent, or any combination thereof.

In another example, the formulation is shelf-stable. Optionally, the shelf-stable formulation is in a dry formulation, a powder formulation, or a lyophilized formulation. In some examples, the formulation is formulated to provide stability for the bacteria. In one example, the formulation is substantially stable at temperatures between about -20 °C and about 50 °C for at least about 1, 2, 3, 4, 5, or 6 days, or 1, 2, 3, or 4 weeks, or 1, 2, 3, 4, 5, 6, 7, 8, 9, 10, 11, or 12 months, or one or more years. In another example, the formulation is substantially stable at temperatures between about 4 °C and about 37 °C for at least about 5, 10, 15, 20, 25, 30, or greater than 30 days.

Bacterial strains produce a plethora of small compounds and secondary metabolites that can be secreted in the culture or be stored endogenously. These may be the microbial active ingredients derived from said bacterial strains. In a particular example, a supernatant from the culture wherein the bacterial strain of the current disclosure has been cultured is utilized, but does not form part of the invention. In another example, an extract or extract fraction from the culture wherein the bacterial strain of current disclosure has been cultured is useful for controlling a pest or pathogen in a plant, but does not form part of the invention. Non-limiting examples of endogenous products are amino acids, peptides, enzymes, secondary metabolites, vitamins, and minerals. In some examples, a metabolite produced by the purified bacterial strain of the present disclosure is contemplated. In some examples, a cell-free or inactivated preparation of the purified bacterial strain of the present disclosure is contemplated, but does not form part of the invention. Removing the cell walls and/or cell membranes of the bacterial strain in culture can be obtained by several procedures which are well-known by the person skilled in the art. Non-limiting examples are the addition of chemicals to said culture, heating said culture or induce lysis in a mechanical way. An extract can also be obtained by autolysis of the bacterial strain. In a preferred example, the microbial active ingredient comprises a spore suspension, spray-dried spores, or a whole cell broth.

Said formulation may be further supplemented with an active ingredient such as a fertilizer, a micronutrient fertilizer material, an insecticide, an herbicide, a plant growth amendment, a fungicide, a molluscicide, an algicide, a bacterial inoculant, a fungal inoculant, or a combination thereof.

In some cases, said fertilizer is a liquid fertilizer. Liquid fertilizer can include without limitation, ammonium sulfate, ammonium nitrate, ammonium sulfate nitrate, ammonium chloride, ammonium bisulfate, ammonium polysulfide, ammonium thiosulfate, aqueous ammonia, anhydrous ammonia, ammonium polyphosphate, aluminum sulfate, calcium nitrate, calcium ammonium nitrate, calcium sulfate, calcined magnesite, calcitic limestone, calcium oxide, hampene (chelated iron), dolomitic limestone, hydrate lime, calcium carbonate, diammonium phosphate, monoammonium phosphate, potassium nitrate, potassium bicarbonate, monopotassium phosphate, magnesium nitrate, magnesium sulfate, potassium sulfate, potassium chloride, sodium nitrates, magnesian limestone, magnesia, disodium dihydromolybdate, cobalt chlorid hexahydrate, nickel chloride hexahydrate, indole butyric acid, L-tryptophan, urea, urea-formaldehydes, urea ammonium nitrate, sulfur-coated urea, polymer-coated urea, isobutylidene diurea, K2SO4-2MgSO4, kainite, sylvinite, kieserite, Epsom salts, elemental sulfur, marl, ground oyster shells, fish meal, oil cakes, fish manure, blood meal, rock phosphate, super phosphates, slag, bone meal, wood ash, manure, bat guano, peat moss, compost, green sand, cottonseed meal, feather meal, crab meal, fish emulsion or a combination thereof. The micronutrient fertilizer material can comprise boric acid, a borate, a boron frit, copper sulfate, a copper frit, a copper chelate, a sodium tetraborate decahydrate, an iron sulfate, an iron oxide, iron ammonium sulfate, an iron frit, an iron chelate, a manganese sulfate, a manganese oxide, a manganese chelate, a manganese chloride, a manganese frit, a sodium molybdate, molybdic acid, a zinc sulfate, a zinc oxide, a zinc carbonate, a zinc frit, zinc phosphate, a zinc chelate or a combination thereof. The insecticide can include an organophosphate, a carbamate, a pyrethroid, an acaricide, an alkyl phthalate, boric acid, a borate, a fluoride, sulfur, a haloaromatic substituted urea, a hydrocarbon ester, a biologically-based insecticide, or a combination thereof. The herbicide can comprise a chlorophenoxy compound, a nitrophenolic compound, a nitrocresolic compound, a dipyridyl compound, an acetamide, an aliphatic acide, an anilide, a benzamide, a benzoic acid, a benzoic acid derivative, anisic acid, an anisic acid derivative, a benzonitrile, benzothiadiazinone dioxide, a thiocarbamate, a carmabate, carbanilate, chloropyridinyl, a cyclohexenone derivative, a dinitroaminobenzene derivative, a fluorodinitrotoluidine compound, isoxazolidinone, nicotinic acide, isopropylamine, an isopropulamine derivative, oxadiazolinone, a phosphate, a phthalate, a picolinic acid compound, a triazine, a triazole, a uracil, a urea derivative, endothall, sodium chlorate, or a combination thereof. The fungicide can comprise a substituted benzene, a thiocarbamate, an ethylene bis dithiocarbamate, a thiophthalidamide, a copper compound, an organomercury compound, an organotin compound, a cadmium compound, anilazine, benomyl, cyclohexamide, dodine, etridiazole, iprodione, metlaxyl, thiamimefon, triforine, or a combination thereof.

Said active ingredient may include other microorganisms, such as said bacterial inoculant or fungal inoculant, preferably which are shown to elicit a beneficiary action to a plant, e.g. pest or pathogen control. More preferably said inoculants also show a positive impact on plant growth and/or yield.

Without wishing to be limitative, said formulation may comprise a fungal inoculant of the family Glomeraceae, a fungal inoculant of the family Claroidoglomeraceae, a fungal inoculant of the family Acaulosporaceae, a fungal inoculant of the family Sacculospraceae, a fungal inoculant of the family Entrophosporaceae, a fungal inoculant of the family Pacidsproraceae, a fungal inoculant of the family Diversisporaceae, a fungal inoculant of the family Paraglomeraceae, a fungal inoculant of the family Archaeosporaceae, a fungal inoculant of the family Geosiphonaceae, a fungal inoculant of the family Ambisporacea, a fungal inoculant of the family Scutellosproaceae, a fungal inoculant of the family Dentiscultataceae, a fungal inoculant of the family Racocetraceae, a fungal inoculant of the phylum Basidiomycota, a fungal inoculant of the phylum Ascomycota, a fungal inoculant of the phylum Zygomycota, a fungal inoculant of the genus Glomus or a combination thereof.

Without wishing to be limitative, said formulation may comprise a bacterial inoculant of genus Rhizobium, bacterial inoculant of the genus Bradyrhizobium, bacterial inoculant of the genus Mesorhizobium, bacterial inoculant of the genus Azorhizobium, bacterial inoculant of the genus Allorhizobium, bacterial inoculant of the genus Burkholderia, bacterial inoculant of the genus Sinorhizobium, bacterial inoculant of the genus Kluyvera, bacterial inoculant of the genus Azotobacter, bacterial inoculant of the genus Pseudomonas, bacterial inoculant of the genus Azosprillium, bacterial inoculant of the genus Bacillus, bacterial inoculant of the genus Streptomyces, bacterial inoculant of the genus Paenibacillus, bacterial inoculant of the genus Paracoccus, bacterial inoculant of the genus Enterobacter, bacterial inoculant of the genus Alcaligenes, bacterial inoculant of the genus Mycobacterium, bacterial inoculant of the genus Trichoderma, bacterial inoculant of the genus Gliocladium, bacterial inoculant of the genus Klebsiella, or a combination thereof.

In one example, said formulation may comprise an agriculturally compatible carrier or excipient. Said "agriculturally compatible carrier" or "agriculturally compatible excipient" which can be regarded as a vehicle, is generally inert and it must be acceptable in agriculture. Thus, the phrase "agriculturally compatible" denotes a substance that can be used routinely under field conditions without interfering with growers' planting equipment, and without adversely influencing crop development or the desired ecological balance in a cultivated area.

The agriculturally compatible carrier or excipient can be solid. Solid carriers or excipients can include but are not limited to clays, natural or synthetic silicates, silica, resins, waxes, solid fertilizers, a polymer, a granular mass, perlite, a perlite granule, peat, a peat pellet, soil, vermiculite, charcoal, sugar factory carbonation press mud, rice husk, carboxymethyl cellulose, fine sand, calcium carbonate, flour, alum, a starch, talc, polyvinyl pyrrolidone, or a combination thereof. The agriculturally compatible carrier or excipient can be a liquid. Liquid carriers or excipients can include but are not limited to water, alcohols, ketones, petroleum fractions, oils, aromatic or paraffinic hydrocarbons, chlorinated hydrocarbons, liquefied gases or a combination thereof. More particularly, the agriculturally compatible carrier or excipient can include a dispersant, a surfactant, an additive, a thickener, an anti-caking agent, residue breakdown, a composting formulation, a granular application, diatomaceous earth, a coloring agent, a stabilizer, a preservative, a polymer, a coating or a combination thereof. One of the ordinary skills in the art can readily determine the appropriate carrier or excipient to be used taking into consideration factors such as a particular bacterial strain, plant to which the inoculum is to be applied, type of soil, climate conditions, whether the inoculum is in liquid, solid or powder form, and the like. The additive can comprise an oil, a gum, a resin, a clay, a polyoxyethylene glycol, a terpene, a viscid organic, a fatty acid ester, a sulfated alcohol, an alkyl sulfonate, a petroleum sulfonate, an alcohol sulfate, a sodium alkyl butane diamate, a polyester of sodium thiobutant dioate, a benzene acetonitrile derivative, a proteinaceous material, or a combination thereof. The proteinaceous material can include a milk product, wheat flour, soybean meal, blood, albumin, gelatin, or a combination thereof. The thickener can comprise a long chain alkylsulfonate of polyethylene glycol, polyoxyethylene oleate or a combination thereof. The surfactant can contain a heavy petroleum oil, a heavy petroleum distillate, a polyol fatty acid ester, a polyethoxylated fatty acid ester, an aryl alkyl polyoxyethylene glycol, an alkyl amine acetate, an alkyl aryl sulfonate, a polyhydric alcolhol, an alkyl phosphate, or a combination thereof. The anti-caking agent can include a sodium salt such as a sodium sulfite, a sodium sulfate, a sodium salt of monomethyl naphthalene sulfonate, or a combination thereof; or a calcium salt such as calcium carbonate, diatomaceous earth, or a combination thereof. The agriculturally compatible carrier or excipient can also include a fertilizer, a micronutrient fertilizer material, an insecticide, a herbicide, a plant growth amendment, a fungicide, a molluscicide, an algicide, a bacterial inoculant, a fungal inoculant, or a combination thereof. Non-limiting examples are provided above.

In one example, the purified bacterial strain or an agricultural active formulation comprising the purified bacterial strain or a lysate derived from the bacterial strain of the present disclosure can be applied to the soil, plant, seed or other plant parts, rhizosphere, or other areas of the plant to which it would be beneficial to apply the agricultural active formulation comprising the purified bacterial strain, agricultural active formulation or microbial active ingredient derived of said bacterial strain of the disclosure.

This disclosure therefore also relates to a plant part coated with the agricultural active formulation comprising the purified bacterial strain or a lysate derived from the bacterial strain.

In a third aspect, the use is described of a purified bacterial strain or the agricultural formulation comprising the purified bacterial strain or a lysate derived from the bacterial strain as disclosed before, to control a pest or pathogen in plants. Preferably said pest or pathogen is a pest or pathogen as described below. Preferably, said plant is selected from the list provided below. More preferably said plant is a crop, most preferably said plant is Triticum, Secale, Hordeum, Avena, grapes, Solanaceae, Cucurbitaceae, soft fruit (e.g. strawberries, blueberries, raspberries, blackberries), stone fruit (e.g. peaches, nectarines), citrus or pome fruit.

In an example, said purified bacterial strain or agricultural active formulation comprising the purified bacterial strain or a lysate derived from the bacterial strain as described before is used in post-harvest treatment of various diseases related to pests or pathogen as described below, on various plants as described below.

"Post-harvest" is defined as the stage of plant or crop production immediately following harvest, including cooling, cleaning, sorting and packing. The instant a crop is removed from the ground, or separated from its parent plant, it begins to deteriorate. Postharvest treatment of a crop largely determines final quality, whether a crop is sold for fresh consumption, or used as an ingredient in a processed food product.

In an example said purified bacterial strain or comprising the purified bacterial strain or a lysate derived from the bacterial strain as described before is used in integrated pest management programs.

"Integrated pest management" (IPM), also known as "integrated pest control" (IPC), is a broad-based approach that integrates practices for economic control of pests. IPM aims to suppress pest populations below the economic injury level (EIL). The UN's Food and Agriculture Organization defines IPM as "the careful consideration of all available pest control techniques and subsequent integration of appropriate measures that discourage the development of pest populations and keep pesticides and other interventions to levels that are economically justified and reduce or minimize risks to human health and the environment. IPM emphasizes the growth of a healthy crop with the least possible disruption to agro-ecosystems and encourages natural pest control mechanisms".

In a final aspect, a method of controlling a pest or pathogen in plants is described. Said method comprises artificially introducing a purified bacterial strain, a bacterial population comprising said bacterial strain, an agriculturally active formulation comprising said bacterial strain or a lysate derived from said bacterial strain to a plant, a plant part or a substrate comprising or hosting said plant, thereby conferring pest or pathogen resistance or pest or pathogen control to said plant, wherein said bacterial strain is a purified bacterial strain as described before, and said agricultural active formulation is an agricultural active formulation as described before.

In one example, said bacterial strain is soil sourced and further propagated outside its original environment.

The current method is particularly useful to be used for monocotyledonous and dicotyledonous plants, including fodder or forage legumes, ornamental plants, food crops, trees or shrubs. Preferably said plant is selected from the list comprising *Acer* spp., *Actinidia* spp., *Abelmoschus* spp., *Agave sisalana, Agropyron* spp., *Agrostis stolonifera, Allium* spp., *Amaranthus* spp., *Ammophila arenaria, Ananas comosus, Annona* spp., *Apium graveolens, Arachis* spp., *Artocarpus* spp., *Asparagus officinalis, Avena* spp. (e.g. *Avena sativa, Avena fatua, Avena byzantina, Avena fatua var. sativa, Avena hybrida*), *Averrhoa carambola, Bambusa* sp., *Benincasa hispida, Bertholletia excelsea, Beta vulgaris, Brassica* spp. (e.g. *Brassica napus, Brassica rapa* ssp. [canola, oilseed rape, turnip rape]), *Cadaba farinosa, Camellia sinensis, Canna indica, Cannabis sativa, Capsicum* spp., *Carex elata, Carica papaya, Carissa macrocarpa, Carya* spp., *Carthamus tinctorius, Castanea* spp., *Ceiba pentandra, Cichorium endivia, Cinnamomum* spp., *Citrullus lanatus, Citrus* spp., *Cocos* spp., *Coffea* spp., *Colocasia esculenta, Cola* spp., *Corchorus* sp., *Coriandrum sativum, Corylus* spp., *Crataegus* spp., *Crocus sativus, Cucurbita* spp., *Cucumis* spp., *Cynara* spp., *Daucus carota, Desmodium* spp., *Dimocarpus longan, Dioscorea* spp., *Diospyros* spp., *Echinochloa* spp., *Elaeis* sp. (e.g. *Elaeis guineensis, Elaeis oleifera*), *Eleusine coracana, Eragrostis tef, Erianthus* sp., *Eriobotrya japonica, Eucalyptus* sp., *Eugenia uniflora, Fagopyrum* spp., *Fagus* spp., *Festuca arundinacea, Ficus carica, Fortunella* spp., *Fragaria* spp., *Ginkgo biloba, Glycine* spp. (e.g. *Glycine max, Soja hispida* or *Soja max*), *Gossypium hirsutum, Helianthus* spp. (e.g. *Helianthus annuus*), *Hemerocallis fulva, Hibiscus* spp., *Hordeum* spp. (e.g. *Hordeum vulgare*), *Ipomoea batatas, Juglans* spp., *Lactuca sativa, Lathyrus* spp., *Lens culinaris, Linum usitatissimum, Litchi chinensis, Lotus* spp., *Luffa acutangula, Lupinus* spp., *Luzula sylvatica, Lycopersicon* spp. (e.g. *Lycopersicon esculentum, Lycopersicon lycopersicum, Lycopersicon pyriforme), Macrotyloma* spp., *Malus* spp., *Malpighia emarginata, Mammea americana, Mangifera indica, Manihot* spp., *Manilkara zapota, Medicago sativa, Melilotus* spp., *Mentha* spp., *Miscanthus sinensis, Momordica* spp., *Morus nigra, Musa* spp., *Nicotiana* spp., *Olea* spp., *Opuntia* spp., *Ornithopus* spp., *Oryza* spp. (e.g. *Oryza sativa, Oryza latifolia*), *Panicum miliaceum, Panicum virgatum, Passiflora edulis, Pastinaca sativa, Pennisetum* sp., *Persea* spp., *Petroselinum crispum, Phalaris arundinacea, Phaseolus* spp., *Phleum pratense, Phoenix* spp., *Phragmites australis, Physalis* spp., *Pinus* spp., *Pistacia vera, Pisum* spp., *Poa* spp., *Populus* spp., *Prosopis* spp., *Prunus* spp., *Psidium* spp., *Punica granatum, Pyrus communis, Quercus* spp., *Raphanus sativus, Rheum rhabarbarum, Ribes* spp., *Ricinus communis, Rubus* spp., *Saccharum* spp., *Salix* sp., *Sambucus* spp., *Secale cereale, Sesamum* spp., *Sinapis* sp., *Solanum* spp. (e.g. *Solanum tuberosum, Solanum integrifolium* or *Solanum lycopersicum*), *Sorghum bicolor, Spinacia* spp., *Syzygium* spp., *Tagetes* spp., *Tamarindus indica, Theobroma cacao, Trifolium* spp., *Tripsacum dactyloides, Triticosecale rimpaui, Triticum* spp. (e.g. *Triticum aestivum, Triticum durum, Triticum turgidum, Triticum hybernum, Triticum macha, Triticum sativum, Triticum monococcum* or *Triticum vulgare*), *Tropaeolum minus, Tropaeolum majus, Vaccinium* spp., *Vicia* spp., *Vigna* spp., *Viola odorata, Vitis* spp., *Zea mays, Zizania palustris, Ziziphus* spp., amongst others; including the progenies and hybrids between the above.

In a preferred example, said plant is chosen from the group of Avena, Axonopus, Buchloe, Coix, Cynodon, Dactylis, Eragrostis, Eremochloa, Festuca, Hordeum, Lolium, Oryza, Paspalum, Pennisetum, Phleum, Poa, Saccharum, Secale, Sorghum, Stenotaphrum, Triticum, xTriticosecala, Zea, Zoysia, grapes, Solanaceae, Cucurbitaceae, soft fruit (e.g. strawberries, blueberries, raspberries, blackberries), stone fruit (e.g. peaches, nectarines), citrus or pome fruit, including the progenies and hybrids between the above.

In a particular preferred example, said plant is *Triticum, Secale, Hordeum, Avena,* grapes, Solanaceae, Cucurbitaceae, soft fruit (e.g. strawberries, blueberries, raspberries, blackberries), stone fruit (e.g. peaches, nectarines), citrus or pome fruit.

The method is particularly useful to control a pest or pathogen in plants as described above. Said pests or pathogen may be any Eukaryotic or Prokaryotic pests or pathogens, such as but not limited to viral, bacterial, fungal pathogens or insect pests. In one example, said pathogens are viral, bacterial or fungal pathogens. In a preferred example, said pests or pathogens are selected from *Phytophthora infestans;* from viral pathogens such as Barley yellow dwarf virus; form bacterial pathogens such as *Xanthomonas* sp. (campestris), *Pseudomonas syringae, Rhizobium* sp.; from fungal pathogens such as *Alternaria solani, Monilinia fructigena, Penicillium digitatum, Penicillium expansum, Penicillium italicum, Tilletia tritici, Claviceps purpurea, Oculimacula* spp., *Fusarium* sp., *Phytium* sp., *Erysiphe graminis, Puccinia graminis, Puccinia triticina, Puccinia striiformis, Pyrenophora tritici-repentis, Ramularia* sp. (collo-cygni), *Sclerotinia sclerotiorum, Botrytis cinerea, Colletotrichum graminicola, Microdochium nivale, Gaeumannomyces graminis var. tritici, Rhizoctonia solani, Tapesia* sp., *Thysanoptera, Ustilago* spp., *Blumeria graminis, Podosphaera xanthii, Mycosphaerella* spp. (i.e. *Zymoseptoria tritici*), *Erysiphe necator, Oidium lycopersicum;* from insect pests such as *Rhopalosuphum padi, Diuraphis noxia, Sitobion avenae, Mythimna unipunctata, Spodoptera praefica, Euschistus* spp., *Aeolus* spp., *Anchastus* spp., *Melanotus* spp., *Limonius* spp., *Lepidoptera, Orthoptera, Coleoptera* or *Hemiptera.*

Methods for introducing bacteria to plants may include: treating the plant and/or a plant part and/or growth medium wherein said plant is grown, with the bacteria or formulation are described herein; an inoculation method comprising adhering bacteria to seeds; a method comprising coating seeds with bacteria; a method comprising directly inoculating bacteria to plants or plant parts; a method of treating (e.g. spraying or dipping) plant parts or whole plants with the bacteria, or formulation described herein. Preferably, a wheat ear, spike, spikelet, stem, leaf, flowers and/or fruits is treated.

An appropriate method may be chosen depending on the type of plant to which the bacteria are to be introduced.

For the purpose of introducing said bacterial strain to said plant or plant parts, the bacterial strain may be formulated as an agricultural active formulation comprising said bacterial strain, or lysate derived from said bacterial strain as active ingredient, said formulation is suitable to be used as a pest or pathogen control agent for plants, said bacterial strain or microbial active ingredient are according to one of the examples as described above.

As a non-limiting example, the isolated bacterial strain or formulation may be dispersed in physiological saline. This solution may be used to coat seeds with the bacterial strain by spraying on the seeds or soaking said seeds in said solution. In another example, for coating seeds with a solution as described in the previous example, a binding agent may be added, such as a binding agent comprising carbide (calcium carbonate). Alternatively, the bacterial strain or a formulation comprising said bacterial strain may be added to a plant or plant parts, e.g. by inoculation, spraying or wetting of said plant or plant parts.

As another of further non-limiting example, the bacterial strain or lysate derived thereof may be applied in the form of coatings or other application. The coating may be applied to a naked and untreated plant part. The coating may be applied as an overcoat to a previously treated plant part. Preferably, the microorganisms are applied in the form of seed coatings or other applications to the seed. Seed coatings are particularly preferred in the treatment of soil borne fungal diseases. The seed coating may be applied to a naked and untreated seed. The seed coating may be applied as a seed overcoat to a previously treated seed. Applying said bacterial strains or microbial consortia to plant parts, like a seed, the plant itself or its substrate modulates a trait of agronomic importance. The trait of agronomic importance can be amongst others pathogen or pest resistance, plant growth, and/or plant yield.

The bacterial strain or microbial active ingredient derived thereof may be applied to the soil or any other substrate in which said plant grows in order to remove pests and/or pathogen from said substrate.

Inoculating the substrate comprising or hosting said plant or plant part can be performed, by way of example and without the intention to be limiting, using a powder, a granule, a pellet, a plug, or a soil drench that is applied to the substrate. Inoculation could also be performed by a liquid application, such as a foliar spray or liquid composition. The application may be applied to a growing plant or to the substrate. Plants, in particular agricultural plants, can be grown in substrate. In one example, said substrate is soil, sand, gravel, polysaccharide, mulch, compost, peat moss, straw, logs, clay, or a combination thereof. In another example, the substrate can also include a hydroculture system or an in vitro culture system. In some examples, a combination of different application methods as described herein is applied. In a non-limiting example, wheat plants are treated against *Fusarium* spp. and/or *Puccinia* spp. by foliar application. Also strawberry plants and tomato plants may for example be treated against *Botrytis* spp. by foliar application. In another non-limiting example, lettuce plants are treated against *Rhizoctonia* spp. and *Pythium* spp. by foliar application, subsequently followed by soil drench applications.

Applying said purified bacterial strain, or an agricultural active formulation comprising the purified bacterial strain or a lysate derived from the bacterial strain to a seed, another plant part, the plant itself, or its substrate modulates a trait of agronomic importance. The trait of agronomic importance will at least be pathogen or pest resistance, and may further include plant growth and/or plant yield.

The invention is further described by the following non-limiting examples which further illustrate the invention, and are not intended to, nor should they be interpreted to, limit the scope of the invention.

### EXAMPLES

The present invention will now be further exemplified with reference to the following examples. The present invention is in no way limited to the given examples or to the embodiments presented in the figures.

### Example 1: Method of storage, cultivation, and formulation of spores of Streptomyces strains according to certain embodiments of the invention

Bacterial strains of the invention, LMG P-32901, B/00314, B/00229, and B/00431, stored at -75 °C in Nutrient Broth (NB) general purpose medium amended with 20% v/v glycerol and were cultured on Nutrient Agar (NA) general purpose medium at 28°C. NA medium is also available from commercial sources, such as Merck KGaA / Sigma-Aldrich (Darmstadt, Germany) product number 70149. For further storage, biomass of the strains was cryopreserved at -20°C. To further propagate the strains, colonies were harvested from the plates and used to inoculate new plates.

For in planta testing of the effect of the bacterial strains on fungal pathogens in climate-controlled growth chambers, living biomass of the bacterial strains of the invention was produced in NB, centrifuged to concentrate the cells, resuspended in an appropriate volume of sterile water with Tween20 for foliar application of spikes of wheat plants by means of dipping or spray application. One day after the application of the biomass of the strains of the invention acting as biocontrol agent (BCA) to the wheat spikes, also the fungal plant pathogen was applied by means of dipping or spray application.

Alternatively, for applying spores of the bacterial strains of the invention instead of living biomass, said spores were produced using solid-state fermentation techniques on an organic carrier, soaked in water with nutrients and additives to enhance sporulation. The spores were separated from the carrier by washing them off in water or water with compounds that serve as a pH buffer or dispersant. The spores were stored at -75°C by supplementing the harvested liquid with 20 v/v% glycerol. The spores were subsequently applied to the wheat spikes using the same preparation procedure as for liquid produced living biomass as described above. Also in this case, the fungal plant pathogen was subsequently applied by means of dipping or spray application.

The effectivity of bacterial strain as a bio-fungicide agent was in both cases determined by the level of necrosis of the plant as compared to an untreated control.

### Mode of action of spore germination

The ability of LMG P-32901 spores' germination and hyphal growth were tested in planta. Wheat spikes and leaves were inoculated with LMG P-32901 spores and incubated up to two weeks at three different temperatures and relative humidities including 20°C, 15°C, and 10°C, and 95%, 90%, 85%, 75%, respectively.

The inoculated spikes and leaves were washed, then the suspensions were analyzed using flow cytometry. Analyzing the flowcytometry results showed that the spores can germinate and produce hyphae one day after inoculation in planta and continue growing up to two weeks at all above mentioned conditions.

### Example 2: In vitro inhibition of fungal plant pathogens of various genera

Purified bacterial strains according to the present invention LMG P-32901, B/00314, B/00229, and B/00431 were used in a co-culturing experiment with fungal (plant) pathogen strains of various genera (Table 1). Solid medium in particular Potato Dextrose Agar (PDA) was prepared and dispensed over petri dishes with a diameter of 8 cm. The purified bacterial strains were cultured in liquid Nutrient Broth until a dense bacterial culture was obtained. 10 µl of the liquid culture was taken and inoculated at 2.5 cm from the center of the petri dish and subsequently incubated at 28 °C overnight. Subsequently, the center of the petri dish was inoculated with 15 µl of a liquid culture of the fungal pathogen and incubated at 21 °C for at least three days.

The inhibiting potential or antifungal activity of the bacterial strains according to the present invention on said fungal pathogens was tested and scored based on the growth inhibition zone or fungal growth radius, measured as from the center of the petri dish. Reference is made to the template shown in Figure 1. Score A indicates that the fungus only grows in the inner circle of the template (fungal radius <10 mm). Score B means that the fungus also grows in the second circle counted from the centre, but does not grown further than the bacterial strain (fungal radius = 10-20 mm). Score C is given when there is an inhibition zone / halo around the bacterial strain, but the fungus can grow past the bacterial strain. Score D means that the fungus can grow up to the bacterial strain, but does not grow over it. A small inhibition zone is visible past the bacterial strain. Finally, score E is given when there is no inhibition of the fungus, and the fungus grows over the bacterial strain. Bacterial strain/fungus combinations which were not tested are indicated with "/".

**Table 1: inhibition scores for four bacterial strains against different pathogenic fungi. Reference is made to the template shown in Figure 1. Score A: The fungus only grows in the inner circle of the template. Fungal radius is <10mm. Score B: The fungus also grows in the second circle but not behind the bacterial strain. Fungal radius = 10-20mm. Score C: There is an inhibition zone / halo around the bacterial strain, but the fungus can grow behind the bacterial strain. Score D: The fungus can grow up to the bacterial strain but does not grow over it. Behind the bacterial strain there is a small inhibition zone. Score E: No inhibition of the fungus, the fungus grows over the bacterial strain. "Not tested" indicates that the combination was not tested.**

| **Pathogen (strain) on PDA** | **LMG P-32901** | **B/00229** | **B/00314** | **B/00431** |
|---|---|---|---|---|
| *Alternaria solani (46816)* | C | D | E | Not tested |
| *Botrytis cinerea (MUCL 399)* | B | A | A | A |
| *Botrytis cinerea (169558)* | B | B | B | Not tested |
| *Botrytis cinerea (169559)* | B | C | B | Not tested |
| *Botrytis cinerea (CBS 810.69)* | B | B | B | Not tested |
| *Fusarium graminearum (BRFM 1995)* | C | D | B | Not tested |
| *Fusarium graminearum (BRFM 1982)* | B | B | B | A |
| *Fusarium graminearum (BRFM 1977)* | B | B | B | Not tested |
| *Fusarium oxysporum (MUCL 39 793)* | B | A | A | A |
| *Microdochium nivale (MUCL18523)* | B | B | A | A |
| *Monilinia fructigena (223846)* | C | B | B | Not tested |
| *Penicilium digitatum (91956)* | A | A | A | A |
| *Pythium sulcatum (CBS 603.73)* | A | A | A | Not tested |
| *Rhizoctonia solani (MUCL 18834)* | B | B | B | Not tested |
| *Sclerotinia sclerotiorum (MUCL 9083)* | B | A | A | A |

Results of the inhibition test indicate a strong inhibitory effect (mostly scores A and B) of the purified bacterial strains LMG P-32901, B/00314, B/00229, and B/00431 on most of the listed fungal plant pathogens. Purified bacterial strain B/00314 showed no clear effect on *Alternaria solani.*

### Example 3: In planta inhibition of fungal pathogen F. graminearum

*F. graminearum* (Fg), also referred to as *Gibberella zeae,* is a fungal plant pathogen causing Fusarium Head Blight (FHB) on wheat and barley. Bacterial strains LMG P-32901, B/00229, B/00431 and B/00314 were used for *in planta* inhibition experiments of the fungus on wheat (*Triticum aestivum*). For the bacterial treatments, flowering spikes of adult wheat plants were treated preventively with the formulated bacterial strains. Two or three days later, the plants were treated with formulated spores (see above) of Fg. A disease control, a formulation control with no pathogen applied later (Mock), and in some experiments a formulation control with the pathogen also applied later (Formulation) were included. After 2 to 3 weeks in a growth chamber, disease severity (as % necrosis) was measured using Fusarium Vision, an in-house developed algorithm linked to an imaging technology for *Fusarium* disease scoring on wheat spikes. Results are shown in Figures 2A-2D. Wheat spikes treated with spores of LMG P-32901, B/00229, B/00431 and B/00314 showed a decrease in disease severity of -72%, -63%, -45% and -54% respectively, compared to the disease control.

### Example 4: In planta inhibition of fungal pathogen Puccinia striiformis var. tritici

*P. striiformis var. tritici* is a plant pathogen causing stripe rust on wheat. In analogy to Example 3, bacterial strains LMG P-32901, and B/00229 and B/00431 were used for *in planta* inhibition experiments of the fungus *P. striiformis* var*. tritici* on wheat leaves. Single leaves were treated with the formulated bacterial strains as a preventive treatment and after a short drying period, the plants were treated with *P. striiformis* var*. tritici* spores. A disease control, for which single leaves were sprayed preventively with *P. striiformis* var*. tritici* spores only, and a formulation control for which the pathogen was later also applied (Formulation) were included. After 2 to 3 weeks days in a growth chamber, disease pressure (as % necrosis) was measured using Puccinia Vision, an in-house developed algorithm linked to an imaging technology for Puccinia disease scoring on wheat leaves. Results are shown in Figure 3.

Results showed a decrease in disease incidence of -76% upon treatment with LMG P-32901, -76% upon treatment with B/00229, and -89% upon treatment with B/00431.

### Example 5: In planta inhibition of fungal pathogen Zymoseptoria tritici

*Z. tritici,* also referred to as *Mycosphaerella graminicola* or *Septoria tritici,* is a fungal wheat plant pathogen causing Septoria Leaf Blotch. In analogy to Example 3 and 4, bacterial strains LMG P-32901, B/00229 and B/00314 were used for *in planta* inhibition experiments of the fungus *Z. tritici* on wheat leaves. For the bacterial treatment, wheat leaves were treated preventively with the bacterial strain. Two to three days later, the leaves were treated with *Z*. *tritici spores.* As a disease control, wheat leaves were treated with *Z. tritici* spores only. Disease severity (as % necrosis) was measured after 3 to 4 weeks in a growth chamber using visual assessment by trained experimenters. Results are shown in Figures 4A-B.

Results showed a decrease in disease severity of -71%, -80% and -68% upon treatment with LMG P-32901, B/00229 and B/00314, respectively, as compared to the disease control.

### Example 6: Reduction of the fungal disease Fusarium Head Blight severity caused by Fusarium sp. in Triticum sp. in field conditions

To evaluate the efficacy of biocontrol agents (bacterial strains of the invention) for Fusarium Head Blight (*F. graminearum*) in wheat, the Findus wheat variety was tested in 24 m² plots in Gietrzwald, Poland. Treatments included two untreated, two mock treatments (formulation without microorganisms), one commercial preventive fungicide treatment (Aviator Xpro), one commercial biocontrol reference treatment (Echiquier) and 4 biocontrol treatments using the bacterial strain LMG P-32901 of the present invention. A properly randomized design was used with 8 replicates for untreated treatment and four replicates for all other treatments.

Artificial inoculation with *F. graminearum* spores was done the evening after the second application of in total two applications by spraying the Fusarium spore solution full field on the trial with a spraying boom (500 l water/ha, 3 bar). In the next 48 hours irrigation was performed.

Biocontrol inoculation was performed in two phases, first at BBCH 61 (10% anthesis), equivalent to the beginning of flowering and the second 2 days later at BBCH63 -65, equivalent to 30-50% anthesis. To assess phytotoxicity, a visual estimation was done 5-7 days after the first application of the treatments. Disease severity was assessed by % of the spike affected by Fusarium, assessed on 50 spikes/plot, as recommended at EPPO guidelines PP1/026(4) - Foliar and ear diseases on cereals. Disease severity was evaluated twice: the first time at +- 15% average disease severity in the untreated and the second time at +- 40% average disease severity in the untreated. All the assessments were performed as recommended in the EPPO guidelines PP1/026(4).

The untreated treatment showed a disease severity of 15.6%. In the treatment where LMG P-32901 was applied, a disease severity of 8.3 % was observed, which was significantly lower (P<0.05 - a reduction of 57.6 %) than the untreated treatment (Figure 5A). The chemical fungicide Aviator Xpro and biological reference treatment Echiquier showed a disease severity of 4% and 6 % respectively (not shown).

A similar experiment using *F. graminearum* spores was repeated on fields in Bulgaria. Results are shown in Figure 5B. Also here, a significant reduction of disease severity (-87.5%) was seen in plants treated with LMG P-32901 compared to untreated controls.

### Example 7: Reduction of the fungal disease caused by Blumeria graminis f. sp. tritici in Triticum sp. in field condition

To evaluate the efficacy of biocontrol agents against powdery mildew (*B. graminis*) in wheat, a field trial was set-up in France. Treatments included three untreated, two mock treatments (formulation without microorganisms), one commercial preventive fungicide treatment (Nissodium), one commercial biocontrol reference treatment (Heliosoufre S) and 8 biocontrol treatments including one using the bacterial strain LMG P-32901 of the present invention. A properly randomized design was used with 12 replicates for untreated treatment and four replicates for all other treatments.

Biocontrol inoculation was performed twice. First, an application was performed when the first disease symptoms were visible on the lower leaf ranks (L4-L5 counting from the bottom). Then, a second application was performed 5 days later.

To assess phytotoxicity, a visual estimation was done 5-7 days after the first application of the treatments. Disease severity was assessed by % of the leaf affected by powdery mildew, assessed on 25 leaves/plot, as recommended at EPPO guidelines PP1/026(4) - Foliar and ear diseases on cereals. Disease severity was evaluated three times: the first time at ≈15% average disease severity in the untreated, the second time at ≈25% average disease severity in the untreated and the third time at ≈50% average disease severity on the untreated. All the assessments were performed as recommended in the EPPO guidelines PP1/026(4). In the treatment where LMG P-32901 was applied, a significant reduction of disease severity was observed (p-val.=0.04), with an observed relative disease reduction of 43.5% (Figure 6A).

Similar experiments using *B. graminis* were performed in fields in Romania and Poland; results are shown in respectively Figures 6B and 6C. A significant relative reduction of disease severity was observed seen in plants treated with LMG P-32901 compared to untreated controls: -83.5% in Romania (p-val.=0.00) and -72.5% in Poland (p-val.=0.00).

### Example 8: Reduction of the fungal disease severity caused by Zymoseptoria tritici in Triticum sp. in field condition

To evaluate the efficacy of biocontrol agents against Septoria leaf blotch (*Z. tritici*) in wheat, a field trial was set-up in Poland. Treatments included two untreated, two mock treatments (formulation without microorganisms), one commercial preventive fungicide treatment (Questar), one commercial biocontrol reference treatment (Heliosoufre S) and 8 biocontrol treatments including one using the bacterial strain LMG P-32901 of the present invention. A properly randomized design was used with 8 replicates for untreated treatment and four replicates for all other treatments.

Biocontrol inoculation was performed twice. First, an application was performed when the average flag leaf had emerged by 50% (BBCH 37). Then, a second application was performed three days later.

To assess phytotoxicity, a visual estimation was done 5-7 days after the first application of the treatments. Disease severity was assessed by % of the leaf affected by powdery mildew, assessed on 25 leaves/plot, as recommended at EPPO guidelines PP1/026(4) - Foliar and ear diseases on cereals. Disease severity was evaluated two times: the first time at ≈15% average disease severity in the untreated, the second time at ≈40-50% average disease severity in the untreated. All the assessments were performed as recommended in the EPPO guidelines PP1/026(4).

In the treatment where LMG P-32901 was applied, a significant reduction of disease severity was observed (p-val.=0.00), with an observed relative disease reduction of 43.7% (Figure 7A).

A similar experiment using *Z. tritici* was performed in fields in Belgium; results are shown in Figure 7B. A relative reduction of disease severity was observed seen in plants treated with LMG P-32901 compared to untreated controls: -16.9% (p-val.=0.09).

Another similar experiment with *Z. tritici* was performed in fields in Germany in 2023; results are shown in Figure 7C. A relative reduction of disease severity was observed in plants treated with LMG P-32901 compared to untreated control: -54.6% (p-val.=0.00).

### Example 9: Reduction of powdery mildew caused by fungal pathogen Podosphaera xanthii in field conditions

To evaluate the efficacy of biocontrol agents, the bacterial strains of the invention, against powdery mildew in cucurbits a trial was set up in an area in the Abruzzo Region in Italy in zucchini, with the variety "Isotta", in open field with 10 m² plots. The area and field were selected to carry out the trial with historical presence and optimal environmental-climatic conditions for the pathogen *Podosphaera xanthii.* Treatments included two untreated, one mock treatment (formulation without microorganisms), one commercial chemical fungicide treatment (Flint Max), one biological reference (Sonata) treatment and 5 biocontrol treatments using the bacterial strain LMG P-32901 of the present invention. The first application was applied preventively and started on the 16th of June, the following 7 applications were applied with an interval of 5 to 7 days.

The efficacy assessments on the leaves (upper and lower leaf surface) were conducted at the disease appearance in all plots, 7 days from the fourth application.

The following assessments were carried out before each application and the last assessment 10 days after the last application according to the EPPO guidelines PP1/57 (3) - Powdery mildews on cucurbits and other vegetables.

The % of leaf area affected on both upper and lower surfaces of at least four leaves of uniform age was assessed on at least eight plants per plots.

The first assessment was performed on 14th July and detected in the untreated treatment about 28% incidence and 4.6% severity as average (on the upper surface of the leaves) in the untreated plots. The disease progressed to 97.5% incidence and 38% severity in the last assessment on 10th August (10 days after last application).

At the assessment of 21^{st} of July, the untreated treatment showed a disease severity of 25.68 %. The treatment where LMG P-32901 was applied showed a disease severity of 9.87 %, which was significantly lower (P<0.01 - a reduction of 55.6 %) than the untreated treatment. Results are shown in Figure 8B. The chemical fungicide Flint Max showed disease severity of 17.73 %, the biological reference Sonata 15.73 % (not shown).

Treatment with LMG P-32901 resulted in a significantly reduced disease severity of -55.6 % compared to untreated controls.

A similar experiment was performed using the fungal pathogen *Podosphaera xanthii* in zucchini on fields in Greece. Results are shown in Figure 8D. Treatment with LMG P-32901 resulted in a significantly reduced disease severity of -63.2 % compared to untreated controls.

A similar experiment with *Podosphaera xanthii* was performed in pumpkin fields in Italy in 2023; results are shown in Figure 8E. A relative reduction of disease severity was observed in pumpkin plants treated with LMG P-32901 compared to untreated control: -82.7% (p-val.=0.00).

### Example 10: Reduction of powdery mildew caused by fungal pathogen Podosphaera xanthii greenhouse conditions

Similar experiments as in examples 8 and 9 were set-up in greenhouses in Italy and Greece respectively, performed on cucumber plants instead of zucchini and pumpkin plants. Results are shown in Figures 8A (Italy) and 8C (Greece).

Treatment with LMG P-32901 resulted in a significantly reduced disease severity of -69.1 % in Italy and -77.6 % % in Greece compared to untreated controls.

A similar experiment with *Podosphaera xanthii* on cucurbits in greenhouse conditions was performed in Spain in 2023; results are shown in Figure 8F. A relative reduction of disease severity was observed in plants treated with LMG P-32901 compared to untreated control: -86.6% (p-val.=0.00062).

### Example 11: Examples 6-10 performed with bacterial strains B/00229, B/00431, and B/00314

Examples 6 - 10 have been repeated with bacterial strains B/00229, B/00431, and B/00314. Also for those strains, a significant reduction in disease severity was shown when treated with the bacterial strains compared to untreated controls (data not shown).

### Example 12: Reduction of grey mold caused by fungal pathogen Botrytis cinerea in field conditions

To evaluate the efficacy of biocontrol agents, the bacterial strains of the invention, against grey mold in table grape a field trial was set up in the region Valencia in Spain, with the variety "Malvasia", in an open field with 26 m² plots.

The area and field were selected to carry out the trial with historical presence and optimal environmental-climatic conditions for the pathogen *Botrytis cinerea.* Treatments included two untreated, one mock treatment (formulation without microorganisms), one commercial chemical fungicide treatment (Switch), one biological reference (Serenade Aso) treatment and 5 biocontrol treatments where of two with the bacterial strain LMG P-32901 of the present invention. The first application was applied preventively at flowering, the following four applications were applied on a given BBCH of the crop as done as a normal practice by the farmer.

The efficacy assessments were conducted at the bunches at the disease appearance in all plots. The following assessments were carried out eight days after the first assessment.

The untreated treatment showed a disease severity of 21.63%. In the treatment where LMG P-32901 was applied, a disease severity of 7.26 % was observed, which was significantly lower (p=0.000 - a reduction of 66.4 %) than the untreated treatment (Figure 9A). The chemical fungicide Switch and the commercial biocontrol reference treatment (Serenade ASO) showed a disease severity of 4.89% and 8.58 % respectively (data not shown).

A similar experiment was performed to evaluate the efficacy of the bacterial strains of the invention for the control of grey mold (*Botrytis cinerea*) in table grape on a field in the region Alicante in Spain following exactly the same protocol as described above. Results are shown in Figure 9B. Also here, a significant reduction of disease severity (-47.8 %, p=0.00) was seen in plants treated with LMG P-32901 compared to untreated controls (Figure 9B). For the untreated treatment a disease severity of 13.53% was noticed. In the treatment where LMG P-32901 was applied, a disease severity of 7.07 % was observed. The chemical fungicide Switch and the commercial biocontrol reference treatment (Serenade ASO) showed a disease severity of 3.89% and 4.06 % respectively (data not shown).

A comparable experiment with *Botrytis cinerea* on wine grape in field conditions was performed in Italy in 2023. Results are shown in Figure 9C. A relative reduction of disease severity was observed in plants treated with LMG P-32901 compared to the untreated control: -92.4% (p-val. =0.00).

### Example 13: Reduction of the fungal disease caused by Sclerotinia minor in lettuce in controlled conditions

To evaluate the activity of biocontrol agents against *Sclerotinia minor* in lettuce, a growth chamber trial was set-up in Italy. Treatments included an untreated control (UTC), a commercial biocontrol reference treatment (Valcure) and a treatment using the bacterial strain LMG P-32901 of the present invention. A properly randomized design was used with 5 replicates (5 pots per replicate) for all treatments.

Biocontrol agents were applied by dipping the lettuce plants for 30 seconds before they were transplanted in soil inoculated with *Sclerotinia minor.*

To assess phytotoxicity, a visual estimation was done at 6 and 11 days after the dipping application of the biocontrol agent. Disease severity was assessed on individual plants using a score ranging from 0-3 with 0: fully healthy, 1: slight symptoms, 2: severe symptoms but capable to recover and 3: too damaged to recover. Disease severity was assessed after 4-6 day and combined scores were analyzed.

In the treatment with LMG P-32901, a significant reduction of disease severity was observed (p-val. = 0.001), with an observed relative decrease of 79.17% (Figure 10). No phytotoxicity was observed. The commercial biocontrol reference treatment Valcure resulted in a 51.04% decrease of the severity score.

### Example 14: Reduction of the fungal disease Yellow Rust severity caused by Puccinia striiformis var. tritici in Triticum sp. in field conditions

To evaluate the efficacy of biocontrol agents (bacterial strains of the invention) for Yellow Rust (*Puccinia striiformis var. tritici*) in wheat, the Miranda wheat variety was tested in 24 m² plots in Sanpetru Mare, Romania. Treatments included an untreated control and one commercial preventive fungicide treatment (Tebustar EW) as reference treatments and 5 biocontrol treatments using the bacterial strain LMG P-32901 of the present invention. A properly randomized design was used with 5 replicates for each treatment.

Biocontrol inoculation was performed twice, first at BBCH 32 and the second time 7 days later. Disease was allowed to spread naturally and no artificial inoculation was performed.

To assess phytotoxicity, a visual estimation was done 7 days after the first and second application of the treatments. Disease severity was assessed by % of the leaf affected by yellow rust, assessed on 25 leaves/plot, as recommended by EPPO guidelines PP1/026(4) - Foliar and ear diseases on cereals. Disease severity was evaluated at least twice: the first time at +- 15% average disease severity in the untreated and the second time at +- 40% average disease severity in the untreated. All the assessments were performed as recommended in the EPPO guidelines PP1/026(4).

The untreated treatment showed a disease severity of 59.5%. In the treatment where LMG P-32901 was applied, a disease severity of 23.8% was observed, which was significantly lower (P<0.05 - a reduction of 60%) than the untreated treatment (Figure 11A). The chemical fungicide Tebustar EW showed a disease severity of 2% (not shown).

A similar experiment with *Puccinia striiformis var. tritici* was performed on fields in Ireland. Results are shown in Figure 11B. Also here, a significant reduction of disease severity (-58.8%) was seen in plants treated with LMG P-32901 compared to untreated controls.

### Example 15: Reduction of grey mold caused by fungal pathogen Botrytis cinerea in greenhouse conditions

To evaluate the efficacy of biocontrol agents (the bacterial strains of the invention) against grey mold (*Botrytis cinerea*) in tomato, a trial was set up in Italy, with the variety "DRW 7723" in a greenhouse with 12 m² plots. Treatments included two untreated, one mock treatment (formulation without microorganisms), one commercial chemical fungicide treatment (Switch), one biological reference (Serenade Aso) treatment and 4 biocontrol treatments, two of which with the bacterial strain LMG P-32901 of the present invention. The first application was applied preventively at the timing that conditions became favorable for grey mold development. The following five applications were applied on a on a weekly basis. Disease was allowed to spread naturally.

Disease assessments were made on weekly basis as soon as first symptoms were observed. Both leaves and fruits were assessed.

Two weeks after the last application, the untreated treatment showed a disease severity of 8.71%. In the treatment where LMG P-32901 was applied, a disease severity of 1.06% was observed, which was significantly lower (p=0.00047 - a reduction of 85%) than the untreated treatment (Figure 12A). The chemical fungicide Switch and the commercial biocontrol reference treatment (Serenade Aso) showed a disease severity of 0.25% and 3.37% respectively (data not shown). Similar effects were observed for Botrytis incidence on the fruits. The untreated control showed an incidence of 18.70% of Botrytis infected fruits. In the treatment where LMG P-32901 was applied, a disease incidence of 11.55% was observed, which was significantly lower (p=0.000 - a reduction of 84.3%) than the untreated treatment (Figure 12B). The chemical fungicide Switch and the commercial biocontrol reference treatment (Serenade Aso) showed a disease incidence of 1.05% and 5.77% respectively (data not shown).

A similar experiment was performed in another greenhouse in Italy on tomato ("variety Eskol") following exactly the same protocol as described above. In this trial only leaf assessments were made. Results are shown in Figure 12C. Also here, a significant reduction of disease severity (-52%, p=0.00949) was seen in plants treated with LMG P-32901 compared to untreated controls (Figure 12C). For the untreated treatment a disease severity of 20.92% was noticed. In the treatment where LMG P-32901 was applied, a disease severity of 10.30 % was observed. The chemical fungicide Switch and the commercial biocontrol reference treatment (Serenade ASO) showed a disease severity of 1.44% and 10.64 % respectively (data not shown).

### Example 16: Reduction of powdery mildew caused by fungal pathogen Uncinula necator in grapevine in field conditions

To evaluate the efficacy of biocontrol agents (the bacterial strains of the invention) against powdery mildew (*Uncinula necator*) in grapevine, a field trial was set up in Spain, with the variety "Mazuelo" in a field. Treatments included one untreated, one commercial fungicide treatment (Cosavet DF) and one biocontrol treatment, with the bacterial strain LMG P-32901 of the present invention. The first application was applied preventively at the timing that conditions became favorable for powdery mildew development. The following applications were applied on a on a weekly basis, up to 12 applications. Disease was allowed to spread naturally.

Disease assessments were made on weekly basis as soon as first symptoms were observed. Both leaves and bunches were assessed.

At 4 days after the 10^{th} application, the untreated treatment showed a disease severity of 70.5% on bunches. In the treatment where LMG P-32901 was applied, disease severity was significantly reduced compared with the untreated treatment (-45.5%; Figure 13A). Similar effects were observed for powdery mildew severity on the leaves. The untreated control showed an average severity of 44.3%. In the treatment where LMG P-32901 was applied, a reduction in severity of -59.2% was observed, which was significantly lower than the untreated treatment (Figure 13B).

A similar experiment was performed in Italy on grapevine ("variety Moscato Bianco") following exactly the same protocol as described above. Results for powdery mildew severity on bunches are shown in Figure 13C. Also here, a significant reduction of disease severity (-65.9%) was seen in plants treated with LMG P-32901 compared to untreated control (67.5% severity) (Figure 13C). In another trial in Italy ("variety Merlot"), a significant reduction of powdery mildew severity (-60.8%) was observed on leaves from plants treated with LMG P-32901 compared to the untreated control (12.9% severity) (Figure 13D).

The present invention is in no way limited to the embodiments described in the examples and/or shown in the figures.

## Claims

1. A purified bacterial strain suitable for controlling a fungal pathogen in a plant, wherein said strain is:
- a strain as deposited with the Belgian Coordinated Collections of Micro-organisms as Deposit ID: LMG P-32901, or
- a strain as deposited with the Polish Collection of Microorganisms as Deposit ID: B/00314, B/00229, or B/00431.

2. The purified bacterial strain according to claim 1, wherein said strain is the bacterial strain as deposited as Deposit ID: LMG P-32901.

3. The purified bacterial strain according to claim 1 or 2, wherein the fungal pathogen is selected from the group consisting of Fusarium graminearum, Zymoseptoria tritici, Puccinia striiformis, Alternaria solani, Botrytis cinerea, Fusarium oxysporum, Microdochium nivale, Monilinia fructigena, Penicilium digitatum, Penicillium expansum, Penicillium italicum, Pythium sulcatum, Rhizoctonia solani, Blumeria graminis, Podosphaera xanthii, Erysiphe necator, Oidium lycopersicum, and Sclerotinia sclerotiorum.

4. An agricultural active formulation comprising the purified bacterial strain according to any one of claims 1-3, or comprising a microbial active ingredient that comprises a spore suspension, spray-dried spores or a whole cell broth derived from said bacterial strain as active ingredient, said formulation is suitable to be used as a fungal pathogen control agent for plants.

5. An agricultural active formulation comprising a lysate derived from the bacterial strain according to any one of claims 1 to 3, wherein the lysate is obtained by a process comprising:
a) growing said bacterial strain in a suitable culture medium under suitable growth conditions;
b) obtaining the lysate by lysing the bacterial strain,
said lysate is suitable to be used as a fungal pathogen control agent for plants.

6. The agricultural active formulation according to any one of claims 4-5, wherein said formulation further comprises an agricultural compatible excipient.

7. A plant part coated with the agricultural active formulation according to any of the preceding claims 4 to 6.

8. Use of the purified bacterial strain according to any one of claims 1 to 3 or the agricultural formulation according to any one of claims 4 to 6, to control a fungal pathogen in plants, wherein said plant is preferably a crop.

9. A method of controlling a fungal pathogen in plants which comprises artificially introducing the purified bacterial strain according to any one of claims 1-3, a bacterial population comprising said bacterial strain, or the agriculturally active formulation according to any one of claims 4-6 to a plant, a plant part or a substrate comprising or hosting said plant, thereby conferring fungal pathogen resistance or fungal pathogen control to said plant.

10. The method of controlling a fungal pathogen in plants according to claim 9, wherein said pathogens are selected from pathogens belonging to Phytophthora infestans, Tilletia tritici, Claviceps purpurea, Oculimacula spp., Fusarium sp., Pytium sp., Erysiphe graminis, Erysiphe necator, Oidium lycopersicum, Puccinia graminis, Puccinia triticina, Puccinia striiformis, Pyrenophora tritici-repentis, Ramularia sp. (collo-cygni), Sclerotinia sclerotiorum, Botrytis cinerea, Rhizoctonia solani, Colletotrichum graminicola, Microdochium nivale, Gaeumannomyces graminis var. tritici, Tapesia sp., Thysanoptera, Ustilago spp. or Mycosphaerella spp., Alternaria solani, Monilinia fructigena, Blumeria graminis, Podosphaera xanthii, Penicillium digitatum, Penicillium expansum, or Penicillium italicum.

11. The method of controlling a fungal pathogen in plants according to any one of claims 9 to 10, wherein said plants are chosen from the group of Acer spp., Actinidia spp., Abelmoschus spp., Agave sisalana, Agropyron spp., Agrostis stolonifera, Allium spp., Amaranthus spp., Ammophila arenaria, Ananas comosus, Annona spp., Apium graveolens, Arachis spp., Artocarpus spp., Asparagus officinalis, Avena spp., Averrhoa carambola, Bambusa sp., Benincasa hispida, Bertholletia excelsea, Beta vulgaris, Brassica spp., Cadaba farinosa, Camellia sinensis, Canna indica, Cannabis sativa, Capsicum spp., Carex elata, Carica papaya, Carissa macrocarpa, Carya spp., Carthamus tinctorius, Castanea spp., Ceiba pentandra, Cichorium endivia, Cinnamomum spp., Citrullus lanatus, Citrus spp., Cocos spp., Coffea spp., Colocasia esculenta, Cola spp., Corchorus sp., Coriandrum sativum, Corylus spp., Crataegus spp., Crocus sativus, Cucurbita spp., Cucumis spp., Cynara spp., Daucus carota, Desmodium spp., Dimocarpus longan, Dioscorea spp., Diospyros spp., Echinochloa spp., Elaeis sp., Eleusine coracana, Eragrostis tef, Erianthus sp., Eriobotrya japonica, Eucalyptus sp., Eugenia uniflora, Fagopyrum spp., Fagus spp., Festuca arundinacea, Ficus carica, Fortunella spp., Fragaria spp., Ginkgo biloba, Glycine spp., Gossypium hirsutum, Helianthus spp., Hemerocallis fulva, Hibiscus spp., Hordeum spp., Ipomoea batatas, Juglans spp., Lactuca sativa, Lathyrus spp., Lens culinaris, Linum usitatissimum, Litchi chinensis, Lotus spp., Luffa acutangula, Lupinus spp., Luzula sylvatica, Lycopersicon spp., Macrotyloma spp., Malus spp., Malpighia emarginata, Mammea americana, Mangifera indica, Manihot spp., Manilkara zapota, Medicago sativa, Melilotus spp., Mentha spp., Miscanthus sinensis, Momordica spp., Morus nigra, Musa spp., Nicotiana spp., Olea spp., Opuntia spp., Ornithopus spp., Oryza spp., Panicum miliaceum, Panicum virgatum, Passiflora edulis, Pastinaca sativa, Pennisetum sp., Persea spp., Petroselinum crispum, Phalaris arundinacea, Phaseolus spp., Phleum pratense, Phoenix spp., Phragmites australis, Physalis spp., Pinus spp., Pistacia vera, Pisum spp., Poa spp., Populus spp., Prosopis spp., Prunus spp., Psidium spp., Punica granatum, Pyrus communis, Quercus spp., Raphanus sativus, Rheum rhabarbarum, Ribes spp., Ricinus communis, Rubus spp., Saccharum spp., Salix sp., Sambucus spp., Secale cereale, Sesamum spp., Sinapis sp., Solanum spp., Sorghum bicolor, Spinacia spp., Syzygium spp., Tagetes spp., Tamarindus indica, Theobroma cacao, Trifolium spp., Tripsacum dactyloides, Triticosecale rimpaui, Triticum spp., Tropaeolum minus, Tropaeolum majus, Vaccinium spp., Vicia spp., Vigna spp., Viola odorata, Vitis spp., Zea mays, Zizania palustris, Ziziphus spp.; including progenies and hybrids between the above.

12. A method of producing a bacterial lysate suitable for controlling a fungal plant pathogen, wherein the method comprises the steps of:
a) growing the purified bacterial strain according to any one of claims 1-3 in a suitable culture medium under suitable growth conditions; and
b) obtaining the lysate by lysing the bacterial strain.

13. A method of producing a bacterial supernatant suitable for controlling a fungal plant pathogen, the method comprising the steps of:
a) growing the purified bacterial strain according to any one of claims 1-3 in a suitable culture medium; and
b) removing the bacterial cells from the culture medium to obtain the supernatant.

14. A method of producing an agricultural active formulation, the method comprising formulating the lysate produced according to the method of claim 12 into an agricultural active formulation.

15. A method of producing an agricultural active formulation, the method comprising the steps of:
a) growing the purified bacterial strain according to any one of claims 1-3 in a suitable culture medium;
b) removing the bacterial cells from the culture medium to obtain the supernatant; and
c) formulating the supernatant produced in step b) into an agricultural active formulation.

## Patentansprüche

1. Gereinigter Bakterienstamm, geeignet zur Bekämpfung eines Pilzerregers bei einer Pflanze, wobei es sich bei dem Stamm um:
- einen Stamm wie bei den Belgian Coordinated Collections of Microorganisms als Deposit ID LMG P-32901 hinterlegt handelt; oder
- einen Stamm wie bei der Polish Collection of Microorganisms als Deposit ID B/00314, B/00229 oder B/00431 hinterlegt handelt.

2. Gereinigter Bakterienstamm nach Anspruch 1, wobei es sich bei dem Stamm um den Bakterienstamm wie als Deposit ID LMG P-32901 hinterlegt handelt.

3. Gereinigter Bakterienstamm nach Anspruch 1 oder 2, wobei der Pilzerreger aus der Gruppe bestehend aus Fusarium graminearum, Zymoseptoria tritici, Puccinia striiformis, Alternaria solani, Botrytis cinerea, Fusarium oxysporum, Microdochium nivale, Monilinia fructigena, Penicillium digitatum, Penicillium expansum, Penicillium italicum, Pythium sulcatum, Rhizoctonia solani, Blumeria graminis, Podosphaera xanthii, Erysiphe necator, Oidium lycopersicum und Sclerotinia sclerotiorum ausgewählt ist.

4. Landwirtschaftliche Wirkformulierung, umfassend den gereinigten Bakterienstamm nach einem der Ansprüche 1-3 oder einen mikrobiellen Wirkstoff, der eine Sporensuspension, sprühgetrocknete Sporen oder eine von dem Bakterienstamm abgeleitete Ganzzellkultur als Wirkstoff umfasst, wobei die Formulierung zur Verwendung als Bekämpfungsmittel gegen Pilzerreger bei Pflanzen geeignet ist.

5. Landwirtschaftliche Wirkformulierung, umfassend ein Lysat, das von dem Bakterienstamm nach einem der Ansprüche 1 bis 3 abgeleitet ist, wobei das Lysat durch ein Verfahren erhalten wird, das Folgendes umfasst:
a) Kultivieren des Bakterienstamms in einem geeigneten Kulturmedium unter geeigneten Wachstumsbedingungen;
b) Gewinnen des Lysats durch Lysieren des Bakterienstamms,
wobei das Lysat zur Verwendung als Bekämpfungsmittel gegen Pilzerreger bei Pflanzen geeignet ist.

6. Landwirtschaftliche Wirkformulierung nach einem der Ansprüche 4-5, wobei die Formulierung ferner einen landwirtschaftlich kompatibelHilfsstoff umfasst.

7. Pflanzenteil, beschichtet mit der landwirtschaftlichen Wirkformulierung nach einem der vorhergehenden Ansprüche 4 bis 6.

8. Verwendung des gereinigten Bakterienstamms nach einem der Ansprüche 1 bis 3 oder der landwirtschaftlichen Formulierung nach einem der Ansprüche 4 bis 6 zur Bekämpfung eines Pilzerregers bei Pflanzen, wobei es sich bei der Pflanze vorzugsweise um eine Kulturpflanze handelt.

9. Verfahren zur Bekämpfung eines Pilzerregers bei Pflanzen, das künstliches Einbringen des gereinigten Bakterienstamms nach einem der Ansprüche 1-3, einer den Bakterienstamm umfassenden Bakterienpopulation oder der landwirtschaftlichen Wirkformulierung nach einem der Ansprüche 4-6 in eine Pflanze, einen Pflanzenteil oder ein die Pflanze umfassendes oder beherbergendes Substrat umfasst, wodurch der Pflanze eine Resistenz gegen Pilzerreger oder eine Bekämpfung von Pilzerregem verliehen wird.

10. Verfahren zur Bekämpfung eines Pilzerregers bei Pflanzen nach Anspruch 9, wobei die Erreger aus Erregern ausgewählt sind, die zu Phytophthora infestans, Tilletia tritici, Claviceps purpurea, Oculimacula spp., Fusarium sp., Pythium sp., Erysiphe graminis, Erysiphe necator, Oidium lycopersicum, Puccinia graminis, Puccinia triticina, Puccinia striiformis, Pyrenophora tritici-repentis, Ramularia sp. (collo-cygni), Sclerotinia sclerotiorum, Botrytis cinerea, Rhizoctonia solani, Colletotrichum graminicola, Microdochium nivale, Gaeumannomyces graminis var. tritici, Tapesia sp., Thysanoptera, Ustilago spp. oder Mycosphaerella spp., Alternaria solani, Monilinia fructigena, Blumeria graminis, Podosphaera xanthii, Penicillium digitatum, Penicillium expansum oder Penicillium italicum gehören.

11. Verfahren zur Bekämpfung eines Pilzerregers bei Pflanzen nach einem der Ansprüche 9 bis 10, wobei die Pflanzen aus der Gruppe Acer spp., Actinidia spp., Abelmoschus spp., Agave sisalana, Agropyron spp., Agrostis stolonifera, Allium spp., Amaranthus spp., Ammophila arenaria, Ananas comosus, Annona spp., Apium graveolens, Arachis spp., Artocarpus spp., Asparagus officinalis, Avena spp., Averrhoa carambola, Bambusa sp., Benincasa hispida, Bertholletia excelsea, Beta vulgaris, Brassica spp., Cadaba farinosa, Camellia sinensis, Canna indica, Cannabis sativa, Capsicum spp., Carex elata, Carica papaya, Carissa macrocarpa, Carya spp., Carthamus tinctorius, Castanea spp., Ceiba pentandra, Cichorium endivia, Cinnamomum spp., Citrullus lanatus, Citrus spp., Cocos spp., Coffea spp., Colocasia esculenta, Cola spp., Corchorus sp., Coriandrum sativum, Corylus spp., Crataegus spp., Crocus sativus, Cucurbita spp., Cucumis spp., Cynara spp., Daucus carota, Desmodium spp., Dimocarpus longan, Dioscorea spp., Diospyros spp., Echinochloa spp., Elaeis sp., Eleusine coracana, Eragrostis tef, Erianthus sp., Eriobotrya japonica, Eucalyptus sp., Eugenia uniflora, Fagopyrum spp., Fagus spp., Festuca arundinacea, Ficus carica, Fortunella spp., Fragaria spp., Ginkgo biloba, Glycine spp., Gossypium hirsutum, Helianthus spp., Hemerocallis fulva, Hibiscus spp., Hordeum spp., Ipomoea batatas, Juglans spp., Lactuca sativa, Lathyrus spp., Lens culinaris, Linum usitatissimum, Litchi chinensis, Lotus spp., Luffa acutangula, Lupinus spp., Luzula sylvatica, Lycopersicon spp., Macrotyloma spp., Malus spp., Malpighia emarginata, Mammea americana, Mangifera indica, Manihot spp., Manilkara zapota, Medicago sativa, Melilotus spp., Mentha spp., Miscanthus sinensis, Momordica spp., Morus nigra, Musa spp., Nicotiana spp., Olea spp., Opuntia spp., Ornithopus spp., Oryza spp., Panicum miliaceum, Panicum virgatum, Passiflora edulis, Pastinaca sativa, Pennisetum sp., Persea spp., Petroselinum crispum, Phalaris arundinacea, Phaseolus spp., Phleum pratense, Phoenix spp., Phragmites australis, Physalis spp., Pinus spp., Pistacia vera, Pisum spp., Poa spp., Populus spp., Prosopis spp., Prunus spp., Psidium spp., Punica granatum, Pyrus communis, Quercus spp., Raphanus sativus, Rheum rhabarbarum, Ribes spp., Ricinus communis, Rubus spp., Saccharum spp., Salix sp., Sambucus spp., Secale cereale, Sesamum spp., Sinapis sp., Solanum spp., Sorghum bicolor, Spinacia spp., Syzygium spp., Tagetes spp., Tamarindus indica, Theobroma cacao, Trifolium spp., Tripsacum dactyloides, Triticosecale rimpaui, Triticum spp., Tropaeolum minus, Tropaeolum majus, Vaccinium spp., Vicia spp., Vigna spp., Viola odorata, Vitis spp., Zea mays, Zizania palustris, Ziziphus spp., einschließlich Nachkommen und Hybriden zwischen den obigen, gewählt sind.

12. Verfahren zur Herstellung eines Bakterienlysats, das zur Bekämpfung eines phytopathogenen Pilzerregers geeignet ist, wobei das Verfahren die folgenden Schritte umfasst:
a) Kultivieren des gereinigten Bakterienstamms nach einem der Ansprüche 1-3 in einem geeigneten Kulturmedium unter geeigneten Wachstumsbedingungen; und
b) Gewinnen des Lysats durch Lysieren des Bakterienstamms.

13. Verfahren zur Herstellung eines bakteriellen Überstands, der zur Bekämpfung eines phytopathogenen Pilzes geeignet ist, wobei das Verfahren die folgenden Schritte umfasst:
a) Kultivieren des gereinigten Bakterienstamms nach einem der Ansprüche 1-3 in einem geeigneten Kulturmedium; und
b) Entfernen der Bakterienzellen aus dem Kulturmedium, um den Überstand zu erhalten.

14. Verfahren zur Herstellung einer landwirtschaftlichen Wirkformulierung, wobei das Verfahren Formulieren des nach dem Verfahren gemäß Anspruch 12 hergestellten Lysats in eine landwirtschaftliche Wirkformulierung umfasst.

15. Verfahren zur Herstellung einer landwirtschaftlichen Wirkformulierung, wobei das Verfahren die folgenden Schritte umfasst:
a) Kultivieren des gereinigten Bakterienstamms nach einem der Ansprüche 1-3 in einem geeigneten Kulturmedium;
b) Entfernen der Bakterienzellen aus dem Kulturmedium, um den Überstand zu erhalten; und
c) Formulieren des in Schritt b) hergestellten Überstands in eine landwirtschaftliche Wirkformulierung.

## Revendications

1. Souche bactérienne purifiée appropriée pour la lutte contre un pathogène fongique dans une plante, dans laquelle ladite souche est :
- une souche telle que déposée auprès des collections coordonnées belges de microorganismes sous l'ID de dépôt : LMG P-32901, ou
- une souche telle que déposée auprès de la Collection polonaise de microorganismes sous l'ID de dépôt : B/00314, B/00229 ou B/00431.

2. Souche bactérienne purifiée selon la revendication 1, dans laquelle ladite souche est la souche bactérienne telle que déposée sous l'ID de dépôt : LMG P-32901.

3. Souche bactérienne purifiée selon la revendication 1 ou 2, dans laquelle le pathogène fongique est choisi dans le groupe constitué par Fusarium graminearum, Zymoseptoria tritici, Puccinia striiformis, Alternaria solani, Botrytis cinerea, Fusarium oxysporum, Microdochium nivale, Monilinia fructigena, Penicillium digitatum, Penicillium expansum, Penicillium italicum, Pythium sulcatum, Rhizoctonia solani, Blumeria graminis, Podosphaera xanthii, Erysiphe necator, Oidium lycopersicum et Sclerotinia sclerotiorum.

4. Formulation agricole active comprenant la souche bactérienne purifiée selon l'une quelconque des revendications 1 à 3, ou comprenant un ingrédient actif microbien qui comprend une suspension de spores, des spores séchées par pulvérisation ou un bouillon cellulaire entier dérivé de ladite souche bactérienne en tant qu'ingrédient actif, ladite formulation étant appropriée pour être utilisée en tant qu'agent de lutte contre un pathogène fongique pour des plantes.

5. Formulation agricole active comprenant un lysat dérivé de la souche bactérienne selon l'une quelconque des revendications 1 à 3, dans laquelle le lysat est obtenu par un procédé comprenant :
a) la culture de ladite souche bactérienne dans un milieu de culture approprié dans des conditions de croissance appropriées ;
b) l'obtention du lysat par lyse de la souche bactérienne,
ledit lysat étant approprié pour être utilisé comme agent de lutte contre un pathogène fongique pour des plantes.

6. Formulation agricole active selon l'une quelconque des revendications 4 à 5, dans laquelle ladite formulation comprend en outre un excipient agricole compatible.

7. Partie de plante enrobée avec la formulation agricole active selon l'une quelconque des revendications 4 à 6 précédentes.

8. Utilisation de la souche bactérienne purifiée selon l'une quelconque des revendications 1 à 3 ou de la formulation agricole selon l'une quelconque des revendications 4 à 6, pour lutter contre un pathogène fongique chez des plantes, dans laquelle ladite plante est de préférence une plante cultivée.

9. Procédé de lutte contre un agent pathogène fongique chez des plantes, qui comprend l'introduction artificielle de la souche bactérienne purifiée selon l'une quelconque des revendications 1 à 3, d'une population bactérienne comprenant ladite souche bactérienne, ou de la formulation agricole active selon l'une quelconque des revendications 4 à 6 dans une plante, une partie de plante ou un substrat comprenant ou hébergeant ladite plante, conférant ainsi une résistance à un pathogène fongique ou une régulation de pathogène fongique à ladite plante.

10. Procédé de lutte contre un pathogène fongique chez des plantes selon la revendication 9, où lesdits pathogènes sont choisis parmi des pathogènes appartenant à Phytophthora infestans, Tilletia tritici, Claviceps purpurea, Oculimacula spp., Fusarium sp., Pythium sp., Erysiphe graminis, Erysiphe necator, Oidium lycopersicum, Puccinia graminis, Puccinia triticina, Puccinia striiformis, Pyrenophora tritici-repentis, Ramularia sp. (collo-cygni), Sclerotinia sclerotiorum, Botrytis cinerea, Rhizoctonia solani, Colletotrichum graminicola, Microdochium nivale, Gaeumannomyces graminis var. tritici, Tapesia sp., Thysanoptera, Ustilago spp. ou Mycosphaerella spp., Alternaria solani, Monilinia fructigena, Blumeria graminis, Podosphaera xanthii, Penicillium digitatum, Penicillium expansum, ou Penicillium italicum.

11. Procédé de lutte contre un pathogène fongique chez des plantes selon l'une quelconque des revendications 9 à 10, dans lequel lesdites plantes sont choisies dans le groupe d'Acer spp., Actinidia spp., Abelmoschus spp., Agave sisalana, Agropyron spp., Agrostis stolonifera, Allium spp., Amaranthus spp., Ammophila arenaria, Ananas comosus, Annona spp., Apium graveolens, Arachis spp., Artocarpus spp., Asparagus officinalis, Avena spp., Averrhoa carambola, Bambusa sp., Benincasa hispida, Bertholletia excelsea, Beta vulgaris, Brassica spp., Cadaba farinosa, Camellia sinensis, Canna indica, Cannabis sativa, Capsicum spp., Carex elata, Carica papaya, Carissa macrocarpa, Carya spp., Carthamus tinctorius, Castanea spp., Ceiba pentandra, Cichorium endivia, Cinnamomum spp., Citrullus lanatus, Citrus spp., Cocos spp., Coffea spp., Colocasia esculenta, Cola spp., Corchorus sp., Coriandrum sativum, Corylus spp., Crataegus spp., Crocus sativus, Cucurbita spp., Cucumis spp., Cynara spp., Daucus carota, Desmodium spp., Dimocarpus longan, Dioscorea spp., Diospyros spp., Echinochloa spp., Elaeis sp., Eleusine coracana, Eragrostis tef, Erianthus sp., Eriobotrya japonica, Eucalyptus sp., Eugenia uniflora, Fagopyrum spp., Fagus spp., Festuca arundinacea, Ficus carica, Fortunella spp., Fragaria spp., Ginkgo biloba, Glycine spp., Gossypium hirsutum, Helianthus spp., Hemerocallis fulva, Hibiscus spp., Hordeum spp., Ipomoea batatas, Juglans spp., Lactuca sativa, Lathyrus spp., Lens culinaris, Linum usitatissimum, Litchi chinensis, Lotus spp., Luffa acutangula, Lupinus spp., Luzula sylvatica, Lycopersicon spp., Macrotyloma spp., Malus spp., Malpighia emarginata, Mammea americana, Mangifera indica, Manihot spp., Manilkara zapota, Medicago sativa, Melilotus spp., Mentha spp., Miscanthus sinensis, Momordica spp., Morus nigra, Musa spp., Nicotiana spp., Olea spp., Opuntia spp., Ornithopus spp., Oryza spp., Panicum miliaceum, Panicum virgatum, Passiflora edulis, Pastinaca sativa, Pennisetum sp., Persea spp., Petroselinum crispum, Phalaris arundinacea, Phaseolus spp., Phleum pratense, Phoenix spp., Phragmites australis, Physalis spp., Pinus spp., Pistacia vera, Pisum spp., Poa spp., Populus spp., Prosopis spp., Prunus spp., Psidium spp., Punica granatum, Pyrus communis, Quercus spp., Raphanus sativus, Rheum rhabarbarum, Ribes spp., Ricinus communis, Rubus spp., Saccharum spp., Salix sp., Sambucus spp., Secale cereale, Sesamum spp., Sinapis sp., Solanum spp., Sorghum bicolor, Spinacia spp., Syzygium spp., Tagetes spp., Tamarindus indica, Theobroma cacao, Trifolium spp., Tripsacum dactyloides, Triticosecale rimpaui, Triticum spp., Tropaeolum minus, Tropaeolum majus, Vaccinium spp., Vicia spp., Vigna spp., Viola odorata, Vitis spp., Zea mays, Zizania palustris, Ziziphus spp. ; y compris les progénitures de ceux-ci et les hybrides entre ceux-ci

12. Procédé de production d'un lysat bactérien approprié pour la lutte contre un pathogène fongique des plantes, dans lequel le procédé comprend les étapes de :
a) culture de la souche bactérienne purifiée selon l'une quelconque des revendications 1 à 3 dans un milieu de culture approprié dans des conditions de croissance appropriées ; et
b) obtention du lysat par lyse de la souche bactérienne.

13. Procédé de production d'un surnageant bactérien approprié pour la lutte contre un pathogène fongique des plantes, le procédé comprenant les étapes de :
a) culture de la souche bactérienne purifiée selon l'une quelconque des revendications 1 à 3 dans un milieu de culture approprié ; et
b) élimination des cellules bactériennes du milieu de culture pour obtenir le surnageant.

14. Procédé de production d'une formulation agricole active, le procédé comprenant la formulation du lysat produit selon le procédé de la revendication 12 en une formulation agricole active.

15. Procédé de production d'une formulation agricole active, le procédé comprenant les étapes de :
a) culture de la souche bactérienne purifiée selon l'une quelconque des revendications 1 à 3 dans un milieu de culture approprié ;
b) élimination des cellules bactériennes du milieu de culture pour obtenir le surnageant ; et
c) formulation du surnageant produit à l'étape b) en une formulation agricole active.
